# EUROPEAN PATENT APPLICATION

(11) **EP 2 338 507 A1**
(43) Date of publication of application: **29.06.2011**
(21) Application number: 10013107.7
(22) Date of filing: 11.03.2002
(51) Int. Cl.: A61K 39/00, A61K 47/00

(54) **Method of treating malignancies through induction of blood immune responses**

(30) Priority: 09.03.2001 US 274679 P
(62) Divisional of application: 02721331.3
(71) Applicant: Baylor Research Institute, Dallas, TX 75204 (US)
(72) Inventor: Banchereau, Jaques, F., Dallas TX 75230 (US); Palucka, Anna, Karolina, Dallas TX 75204 (US); Fay, Joseph, Dallas TX 75230 (US); Steinman, Ralph, Westport CT 06880 (US); Dhodapkar, Madhav, New Haven CT 06515 (US); Wittkowski, Knut, M., New York NY 10021 (US)
(74) Representative: UEXKÜLL & STOLBERG

(57) **Abstract**

The invention pertains to a vaccine comprising antigen-presenting cells loaded with at least two or more agents selected from the group consisting of tumor antigens and tumor antigen derived peptides and with control antigens, and its use for the treatment and prevention of malignancies.

## Description

### CROSS-REFERENCE TO RELATED APPLICATION

This application claims the benefit of U.S. provisional patent application No. 60/274,679 filed March 9, 2001.

### TECHNICAL FIELD OF INVENTION

This invention relates to the elicitation of blood immune responses including vaccine specific ones as a way to treat malignancies.

### BACKGROUND

Molecular identification of human cancer antigens in the last decade (Knuth, et al. 1989. "Cytolytic T-cell clones against an autologous human melanoma: specificity study and definition of three antigens by immunoselection," Proc Natl Acad Sci USA 86:2804-2808; van der Bruggen, et al. 1991. "A gene encoding an antigen recognized by cytolytic T lymphocytes on a human melanoma," Science 254:1643-1647; and Rosenberg, S. A. 1991. "Cancer vaccines based on the identification of genes encoding cancer regression antigens," Immunol Today 18:175-182) has ushered in a new era of antigen-specific cancer immunotherapy specifically targeting these antigens (Celluzzi, et al. 1996. "Peptide-pulsed dendritic cells induce antigen-specific CTL-mediated protective tumor immunity," JExp Med 183:283-287; Zitvogel, et al. 1996. "Therapy of murine tumors with tumor peptide-pulsed dendritic cells: dependence on T cells, B7 costimulation, and T helper cell 1-associated cytokines," JExp Med 183:87-97; Sogn, J. A. 1998. "Tumor immunology: the glass is half full," Immunity 9:757-763; and Gilboa, E. 1999. "The makings of a tumor rejection antigen," Immunity 11: 263-270). However, several such approaches (e.g. peptides, DNA vaccines and viral vectors) have thus far met with little or no success in actual patient therapy (Gilboa, E. 1999. Immunity 11: 263-270; Pardoll, D. M. 1998. "Cancer vaccines," Nat Med 4:525-531; and Fong, L. and Engleman, E. G. 2000. "Dendritic cells in cancer immunotherapy," Annu Rev Immunol 18:245-273). In particular, it has proven difficult to immunize humans simultaneously with multiple tumor antigens.

Dendritic cells (DCs) are antigen-presenting cells specialized to initiate and regulate immune responses (Steinman, R. M. 1991. "The dendritic cell system and its role in immunogenicity," Annu Rev Immunol 9:271-296; and Banchereau, et al. 2000. "Immunobiology of dendritic cells," Ann Rev Immunol 18:767)*.* Their clinical use as adjuvants has been aided by the development of methodologies to generate large numbers of these cells in culture from blood monocytes (Romani, et al. 1994. "Proliferating dendritic cell progenitors in human blood," JExp Med 180:83-93; and Sallusto, F. and Lanzavecchia, A. 1994. "Efficient presentation of soluble antigen by cultured human dendritic cells is maintained by granulocyte/macrophage colony-stimulating factor plus interleukin 4 and downregulated by tumor necrosis factor alpha," J Exp Med 179:1109-1118) or CD34+ progenitors (Caux, et al. 1992. "GM-CSF and TNF-alpha cooperate in the generation of dendritic Langerhans cells," Nature 360:258-261). U.S. Patent No. 6,004,807 teaches generation of dendritic cells from CD34 hematopoietic progenitors using tissue culture medium supplemented with 10%(v/v) heat inactivated fetal bovine serum. In animal models, TAA-loaded DC have been reported to induce protective/rejection antitumor responses (Boczkowski, et al. 1996. "Dendritic cells pulsed with RNA are potent antigen-presenting cells in vitro and in vivo," J Exp Med 184:465-472; Flamand, et al. 1994 "Murine dendritic cells pulsed in vitro with tumor antigen induce tumor resistance in vivo," Eur J Immunol 24:605-610; Mayordomo, et al. 1995. "Bone marrow-derived dendritic cells pulsed with synthetic tumour peptides elicit protective and therapeutic antitumour immunity," Nat Med 1:1297-1302; Mayordomo, et al. 1997. "Bone marrow-derived dendritic cells serve as potent adjuvants for peptide-based antitumor vaccines," Stem Cells 15:94-103; Porgador, A. and Gilboa, E. 1995. "Bone marrow-generated dendritic cells pulsed with a class I-restricted peptide are potent inducers of cytotoxic T lymphocytes," JExp Med 182:255-260; Song, et al. 1997. "Dendritic cells genetically modified with an adenovirus vector encoding the cDNA for a model antigen induce protective and therapeutic antitumor immunity," J Exp Med 186:1247-1256; Specht, et al. 1997. "Dendritic cells retrovirally transduced with a model antigen gene are therapeutically effective against established pulmonary metastases," J Exp Med 186:1213-1221; and Toes, et al. 1996. "Protective antitumor immunity induced by immunization with completely allogeneic tumor cells," Cancer Res 56:3782-3787).

A number of ongoing and reported clinical trials have utilized TAA-loaded DC as a vaccine in human cancer (Gilboa, E. 1999. Immunity 11:263-270; Timmerman, J. M. and Levy, R. 1999. "Dendritic cell vaccines for cancer immunotherapy," Annu Rev Med 50:507-529; Palucka, et al. 1999. "Dendritic cells and tumor immunity," Curr Opin OEM Drugs*;* Gilboa, et al. 1998. "Immunotherapy of cancer with dendritic-cell-based vaccines," Cancer Immunol Immunother 46:82-87; Bell, et al. 1999. "Dendritic cells," Advances in Immunology 72:255-324; and Banchereau, et al. 2000. "Immunobiology of dendritic cells," Ann Rev Immunol 18:767-812). Some responses have been reported in preliminary trials including: (i) a pioneer study based on injection of blood-derived DC loaded with lymphoma idiotype (Hsu, et al. 1996. "Vaccination of patients with B-cell lymphoma using autologous antigen- pulsed dendritic cells," Nat Med 2:52-58); (ii) administration of peptide-pulsed APC generated by culturing monocytes with GM-CSF (Mukherji, et al. 1995. "Induction of antigen-specific cytolytic T cells in situ in human melanoma by immunization with synthetic peptide-pulsed autologous antigen presenting cells," Proc Natl Acad Sci USA 92:8078-8082); (iii) vaccination with monocyte-derived DC loaded with melanoma peptides (Nestle, et al. 1998. "Vaccination of melanoma patients with peptide- or tumor lysate-pulsed dendritic cells," Nat Med 4:328-332; and Thurner, et al. 1999. "Vaccination with mage-3A1 peptide-pulsed mature, monocyte-derived dendritic cells expands specific cytotoxic T cells and induces regression of some metastases in advanced stage IV melanoma," JExp Med 190:1669-1678); and (iv) injection of monocyte-derived DC pulsed with prostate-specific membrane antigen (PSMA) peptide (Salgaller, et al. 1998 "Report of immune monitoring of prostate cancer patients undergoing T- cell therapy using dendritic cells pulsed with HLA-A2-specific peptides from prostate-specific membrane antigen (PSMA)," Prostate 35:144-151).

The determination of vaccine efficacy constitutes one of the most difficult parameters in immunotherapy protocols. While the ultimate efficacy should be measured by the rate of tumor regression, duration of disease-free survival, or, at least, time-to-disease progression, these end-points require sufficiently long follow-up. Thus, several years of clinical studies based on these efficacy requirements are necessary to conclude whether a given immunotherapy approach is relevant Hence, there is an ongoing search for so called "surrogate markers" that would permit early measurements and be predictive of clinical outcome. As of today, in vitro measurements of tumor-and/or vaccine-specific immune responses have not proven successful as surrogate markers (reviewed in Srivastava, P. 2000. "Immunotherapy of human cancer: lessons from mice," Nat Immunol 1:363-366). Furthermore, responses measured in the most easily accessible organ such as blood are considered irrelevant. Indeed, in a majority of studies, there is a large discrepancy between the elicited immune responses and clinical responses, and hardly any correlations are found. These observations prompted development of alternative methods such as biopsies of tumor lesions to follow the presence of T cells specific for tumor antigens as well as tumor regression. Such approaches, however, are limited to accessible lesions as well as to few medical centers able to perform genomic analyses. Furthermore, the isolated T cells routinely require ex vivo expansion and laborious technology to evaluate their specificity and functional capacity. Thus, such methodologies are not useful for large scale clinical trials and are unlikely to allow early conclusions for non-successful approaches.

A method has now been found to treat malignancy based on the relationship of the elicitation of blood immune responses, including vaccine-specific immune responses, to clinical response. Measuring a patient's overall tumor immunity score over time provides a means for timely predicting efficacy of treatment and monitoring the patient's clinical outcome.

### SUMMARY OF THE INVENTION

In one aspect the present invention is a method for treating malignancy in a mammal comprising administering to the mammal in need of such treatment an effective amount of a vector for vaccination comprising antigen-presenting cells loaded with at least two or more agents selected from the group consisting of tumor antigens and tumor antigen derived peptides. In one embodiment, the antigen-presenting cells are dendritic cells, preferably dendritic cells derived from CD34+ hematopoietic progenitors. The dendritic cells can be generated by culturing CD34 hematopoietic progenitors in a tissue culture in the absence of any foreign serum component. The dendritic cells can also be generated by culturing CD34 hematopoietic progenitors in a tissue culture in the presence of autologous serum, the autologous serum not being heat inactivated before addition to the tissue culture. In this method, the vector for vaccination can also further comprise antigen-presenting cells loaded with control antigens.

In another aspect, the present invention is a method for treating malignancy in a mammal comprising administering to the mammal in need of such treatment an effective amount of a vector for vaccination comprising at least two or more tumor antigens or tumor antigen derived peptides, wherein the vaccine targets antigen-presenting cells in the mammal. In this method, the vector for vaccination can also further comprise antigen-presenting cells loaded with control antigens.

In another aspect, the present invention is a method for treating malignancy in a mammal comprising administering to the mammal in need of such treatment an effective amount of two or more vectors for vaccination wherein each vector comprises antigen-presenting cells loaded with one unique agent selected from the group consisting of tumor antigens and tumor antigen derived peptides. In one embodiment, the antigen-presenting cells are dendritic cells, preferably dendritic cells derived from CD34+ hematopoietic progenitors. The dendritic cells can be generated by culturing CD34 hematopoietic progenitors in a tissue culture in the absence of any foreign serum component. The dendritic cells can also be generated by culturing CD34 hematopoietic progenitors in a tissue culture in the presence of autologous serum, the autologous serum not being heat inactivated before addition to the tissue culture. In this method, the vector for vaccination can also further comprise antigen-presenting cells loaded with control antigens.

In another aspect, the present invention is a method for treating malignancy in a mammal comprising administering to the mammal in need of such treatment an effective amount of two or more vectors for vaccination wherein each vector comprises one unique agent selected from the group consisting of tumor antigens and tumor antigen derived peptides, and wherein the vector targets antigen-presenting cells in the mammal. In this method, the vector for vaccination can also further comprise antigen-presenting cells loaded with control antigens.

In another aspect, the present invention is a method for preventing malignancy in a mammal comprising administering to the mammal an effective amount of a vector for vaccination comprising antigen-presenting cells loaded with at least two tumor antigens or tumor antigen derived peptides. In one embodiment, the antigen-presenting cells are dendritic cells, preferably dendritic cells derived from CD34+ hematopoietic progenitors. The dendritic cells can be generated by culturing CD34 hematopoietic progenitors in a tissue culture in the absence of any foreign serum component. The dendritic cells can also be generated by culturing CD34 hematopoietic progenitors in a tissue culture in the presence of autologous serum, the autologous serum not being heat inactivated before addition to the tissue culture. In this method, the vector for vaccination can also further comprise antigen-presenting cells loaded with control antigens.

In another aspect,'the present invention is a method for preventing malignancy in a mammal comprising administering to the mammal an effective amount of a vector for vaccination comprising at least two or more tumor antigens or tumor antigen derived peptides, wherein the vector targets antigen-presenting cells in the mammal. In this method, the vector for vaccination can also further comprise antigen-presenting cells loaded with control antigens.

In another aspect, the present invention is a method for preventing malignancy in a mammal comprising administering to the mammal an effective amount of two or more vectors for vaccination wherein each vector comprises antigen-presenting cells loaded with one unique agent selected from the group consisting of tumor antigens and tumor antigen derived peptides. In one embodiment, the antigen-presenting cells are dendritic cells, preferably dendritic cells derived from CD34+ hematopoietic progenitors. The dendritic cells can be generated by culturing CD34 hematopoietic progenitors in a tissue culture in the absence of any foreign serum component. The dendritic cells can also be generated by culturing CD34 hematopoietic progenitors in a tissue culture in the presence of autologous serum, the autologous serum not being heat inactivated before addition to the tissue culture. In this method, the vector for vaccination can also further comprise antigen-presenting cells loaded with control antigens.

In another aspect, the present invention is a method for preventing malignancy in a mammal comprising administering to the mammal an effective amount of two or more vectors for vaccination wherein each vector comprises one unique agent selected from the group consisting of tumor antigens and tumor antigen derived peptides, wherein the vector targets antigen-presenting cells in the mammal. In this method, the vector for vaccination can also further comprise antigen-presenting cells loaded with control antigens.

In another aspect, the present invention is a method for predicting the in vivo anti-tumor efficacy of a vaccine, the vaccine comprising antigen-presenting cells loaded with at least two or more tumor antigens, tumor antigen derived peptides or control antigens, comprising isolating at a first time point a first sample of peripheral blood mononuclear cells from a patient before administering the vaccine to form pre-immunization cells; storing the pre-immunization cells under conditions that preserve the proliferative integrity of the pre-immunization cells; immunizing the patient with the vaccine; isolating at at least one subsequent time point a post-immunization sample of peripheral blood mononuclear cells from the patient to form post-immunization cells; separately and simultaneously culturing the pre-immunization and post-immunization cells in the absence of exogenous antigens; measuring the amount of proliferation of the pre-immunization cells and the post-immunization cells; comparing the amount of proliferation of the post-immunization cells to the amount of proliferation of the pre-immunization cells, wherein a ratio of the amount of proliferation of post-immunization cells to the amount of proliferation of pre-immunization cells being greater than or equal to one is positively predictive of effective anti-tumor activity of the vaccine in the patient. In one embodiment, the antigen-presenting cells are dendritic cells, preferably dendritic cells derived from CD34+ hematopoietic progenitors. The dendritic cells can be generated by culturing CD34 hematopoietic progenitors in a tissue culture in the absence of any foreign serum component. The dendritic cells can also be generated by culturing CD34 hematopoietic progenitors in a tissue culture in the presence of autologous serum, the autologous serum not being heat inactivated before addition to the tissue culture.

In another aspect, the present invention is a method for predicting the in vivo anti-tumor efficacy of a vaccine, the vaccine comprising two or more vectors wherein each vector comprises antigen-presenting cells loaded with one unique agent selected from the group consisting of tumor antigens, tumor antigen derived peptides and control antigens, comprising isolating at a first time point a first sample of peripheral blood mononuclear cells from a patient before administering the vaccine to form pre-immunization cells; storing the pre-immunization cells under conditions that preserve the proliferative integrity of the pre-immunization cells; immunizing the patient with the vaccine; isolating at at least one subsequent time point a post-immunization sample of peripheral blood mononuclear cells from the patient to form post-immunization cells; separately and simultaneously culturing the pre-immunization and post-immunization cells in the absence of exogenous antigens; measuring the amount of proliferation of the pre-immunization cells and the post-immunization cells; comparing the amount of proliferation of the post-immunization cells to the amount of proliferation of the pre-immunization cells, wherein a ratio of the amount of proliferation of post-immunization cells to the amount of proliferation of pre-immunization cells being greater than or equal to one is positively predictive of effective anti-tumor activity of the vaccine in the patient. In one embodiment, the antigen-presenting cells are dendritic cells, preferably dendritic cells derived from CD34+ hematopoietic progenitors. The dendritic cells can be generated by culturing CD34 hematopoietic progenitors in a tissue culture in the absence of any foreign serum component. The dendritic cells can also be generated by culturing CD34 hematopoietic progenitors in a tissue culture in the presence of autologous serum, the autologous serum not being heat inactivated before addition to the tissue culture.

In another aspect, the present invention is a method for predicting the in vivo anti-tumor efficacy of a vaccine, the vaccine comprising at least two or more vectors of vaccination wherein each vector comprises one unique agent selected from the group consisting of tumor antigens, tumor antigen derived peptides and control antigens, wherein the vectors target antigen-presenting cells in the mammal, comprising isolating at a first time point a first sample of peripheral blood mononuclear cells from a patient before administering the vaccine to form pre-immunization cells; storing the pre-immunization cells under conditions that preserve the proliferative integrity of the pre-immunization cells; immunizing the patient with the vaccine; isolating at at least one subsequent time point a post-immunization sample of peripheral blood mononuclear cells from the patient to form post-immunization cells; separately and simultaneously culturing the pre-immunization and post-immunization cells in the absence of exogenous antigens; measuring the amount of proliferation of the pre-immunization cells and the post-immunization cells; comparing the amount of proliferation of the post-immunization cells to the amount of proliferation of the pre-immunization cells, wherein a ratio of the amount of proliferation of post-immunization cells to the amount of proliferation of pre-immunization cells being greater than or equal to one is positively predictive of effective anti-tumor activity of the vaccine in the patient.

In another aspect, the present invention is a method for predicting the in vivo anti-tumor efficacy of a vaccine, the vaccine comprising a vector for vaccination comprising at least two or more tumor antigens, tumor antigen derived peptides or control antigens, wherein the vector targets antigen-presenting cells in the mammal, comprising isolating at a first time point a first sample of peripheral blood mononuclear cells from a patient before administering the vaccine to form pre-immunization cells; storing the pre-immunization cells under conditions that preserve the proliferative integrity of the pre-immunization cells; immunizing the patient with the vaccine; isolating at at least one subsequent time point a post-immunization sample of peripheral blood mononuclear cells from the patient to form post-immunization cells; separately and simultaneously culturing the pre-immunization and post-immunization cells in the absence of exogenous antigens; measuring the amount of proliferation of the pre-immunization cells and the post-immunization cells; comparing the amount of proliferation of the post-immunization cells to the amount of proliferation of the pre-immunization cells, wherein a ratio of the amount of proliferation of post-immunization cells to the amount of proliferation of pre-immunization cells being greater than or equal to one is positively predictive of effective anti-tumor activity of the vaccine in the patient.

In one aspect, the present invention is a method for predicting the in vivo anti-tumor efficacy of a vaccine, the vaccine comprising antigen-presenting cells loaded with at least two or more agents selected from the group consisting of tumor antigens, tumor antigen derived peptides and control antigens, comprising immunizing at a first time point a patient with the vaccine; measuring at at least one post-immunization time point the patient's immunologic response to the agents; wherein a positive immunologic response to at least two agents is positively predictive of effective anti-tumor activity of the vaccine in the patient. In one embodiment, the antigen-presenting cells are dendritic cells, preferably dendritic cells derived from CD34+ hematopoietic progenitors. The dendritic cells can be generated by culturing CD34 hematopoietic progenitors in a tissue culture in the absence of any foreign serum component. The dendritic cells can also be generated by culturing CD34 hematopoietic progenitors in a tissue culture in the presence of autologous serum, the autologous serum not being heat inactivated before addition to the tissue culture.

In another aspect, the present invention is a method for predicting the in vivo anti-tumor efficacy of a vaccine, the vaccine comprising two or more vectors wherein each vector comprises antigen-presenting cells loaded with one unique agent selected from the group consisting of tumor antigens, tumor antigen derived peptides and control antigens, comprising immunizing at a first time point a patient with the vaccine; measuring at at least one post-immunization time point the patient's immunologic response to the agents; wherein a positive immunologic response to at least two agents is positively predictive of effective anti-tumor activity of the vaccine in the patient. In one embodiment, the antigen-presenting cells are dendritic cells, preferably dendritic cells derived from CD34+ hematopoietic progenitors. The dendritic cells can be generated by culturing CD34 hematopoietic progenitors in a tissue culture in the absence of any foreign serum component. The dendritic cells can also be generated by culturing CD34 hematopoietic progenitors in a tissue culture in the presence of autologous serum, the autologous serum not being heat inactivated before addition to the tissue culture.

In another aspect, the present invention is a method for predicting the in vivo anti-tumor efficacy of a vaccine, the vaccine comprising a vector for vaccination comprising at least two or more agents selected from the group consisting of tumor antigens, tumor antigen derived peptides and control antigens, comprising immunizing at a first time point a patient with the vaccine; measuring at at least one post-immunization time point the patient's immunologic response to the agents, wherein a positive immunologic response to at least two agents is positively predictive of effective anti-tumor activity of the vaccine in the patient.

In another aspect, the present invention is a method for predicting the in vivo anti-tumor efficacy of a vaccine, the vaccine comprising two or more vectors wherein each vector one unique agent selected from the group consisting of tumor antigens, tumor antigen derived peptides and control antigens, comprising immunizing a patient with the vaccine; measuring at at least one post-immunization time point the patient's immunologic response to the agents, wherein a positive immunologic response to at least two agents is positively predictive of effective anti-tumor activity of the vaccine in the patient.

In another aspect, the present invention is a method for predicting a subject's clinical outcome from two or more immune responses, comprising identifying the subject as a separate group to be compared with previously treated subjects grouped by their clinical response; partially ordering all profiles by determining for all pairs of profiles the order of a first profile compared to a second profile as superior, inferior, equal, or undecided, wherein a partial ordering comprises the first profile superior if for each variable the first profile is superior or equal, and for at least one variable, the first profile is superior; computing all rankings compatible with all pairwise partial orderings, wherein among two ordered subjects the subject being superior is assigned the higher rank; generating a score for each profile by averaging across the rankings; and predicting the clinical outcome by selecting the group whose average score most closely resembles the score of the subject's profile.

In another aspect, the present invention is a method for generating CD34 dendritic cells, comprising culturing CD34 hematopoietic progenitors in a tissue culture in the absence of any foreign serum component.

In another aspect, the present invention is a method for generating CD34 dendritic cells, comprising culturing CD34 hematopoietic progenitors in a tissue culture in the presence of autologous serum, the autologous serum not being heat inactivated before addition to the tissue culture.

In yet another aspect, the present invention is a vaccine comprising antigen-presenting cells loaded with at least two or more agents selected from the group consisting of tumor antigens, tumor antigen derived peptides and control antigens, wherein the antigen-presenting cells are CD34 dendritic cells isolated from a tissue culture of CD34 hematopoietic progenitors wherein the a tissue culture lacks any foreign serum component.

In yet another aspect, the present invention is a vaccine comprising antigen-presenting cells loaded with at least two or more agents selected from the group consisting of tumor antigens, tumor antigen derived peptides and control antigens, wherein the antigen-presenting cells are CD34 dendritic cells isolated from a tissue culture of CD34 hematopoietic progenitors wherein the tissue culture comprises autologous serum, the autologous serum not being heat inactivated before addition to the tissue culture.

In yet another aspect, the invention is a vaccine comprising two or more vectors for vaccination wherein each vector comprises antigen-presenting cells loaded with one unique agent selected from the group consisting of tumor antigens, tumor antigen derived peptides and control antigens, wherein the antigen-presenting cells are CD34 dendritic cells isolated from a tissue culture of CD34 hematopoietic progenitors wherein the tissue culture lacks any foreign serum component.

In yet another aspect, the present invention is a vaccine comprising two or more vectors for vaccination wherein each vector comprises antigen-presenting cells loaded with one unique agent selected from the group consisting of tumor antigens, tumor antigen derived peptides and control antigens, wherein the antigen-presenting cells are CD34 dendritic cells isolated from a tissue culture of CD34 hematopoietic progenitors wherein the tissue culture comprises autologous serum, the autologous serum not being heat inactivated before addition to the tissue culture.

In yet another aspect, the present invention is a vaccine comprising at least two or more vectors selected from the group consisting of tumor antigens, tumor antigen derived peptides and control antigens targeting antigen-presenting cells in a mammal.

### BRIEF DESCRIPTION OF THE DRAWINGS

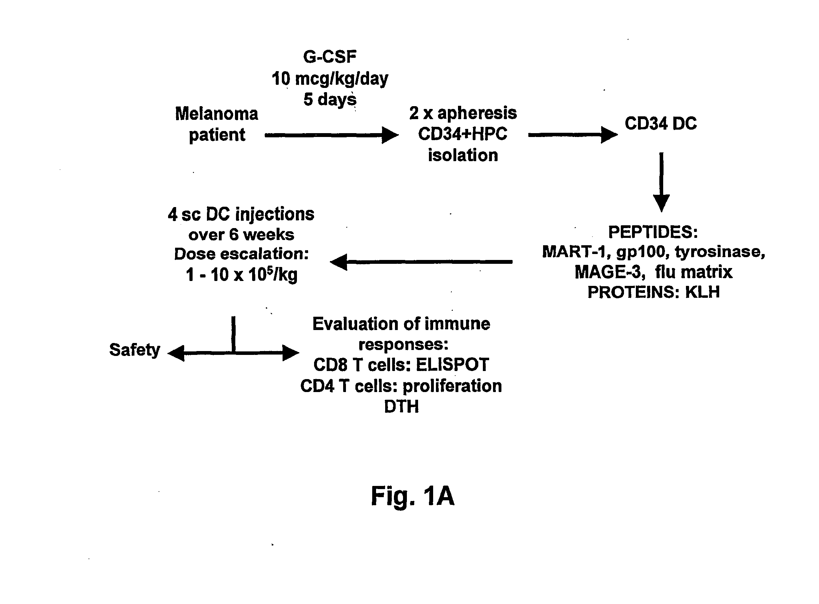

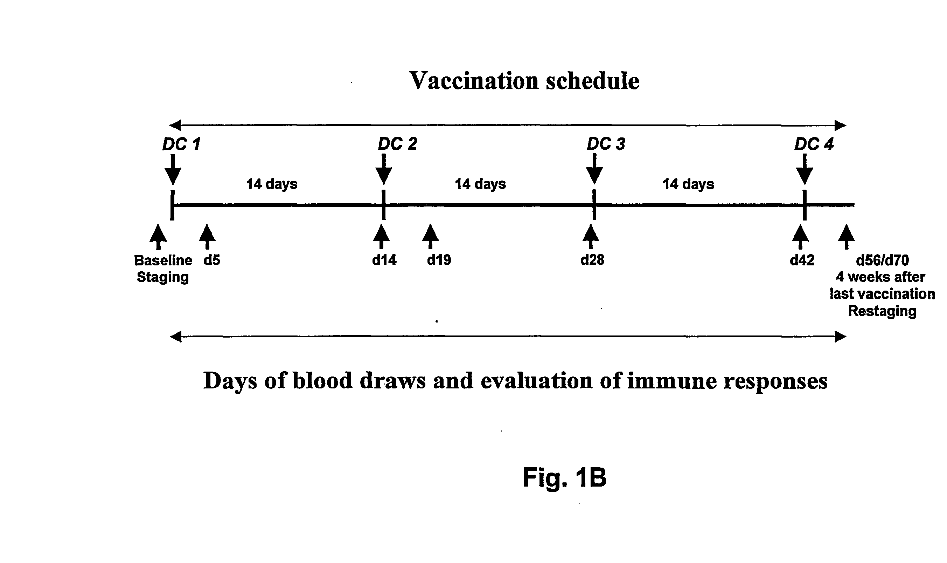

Fig. 1A and 1B summarize the clinical study presented herein. Fig. 1A depicts study design and Fig. 1B, the vaccination and blood draw schedules.

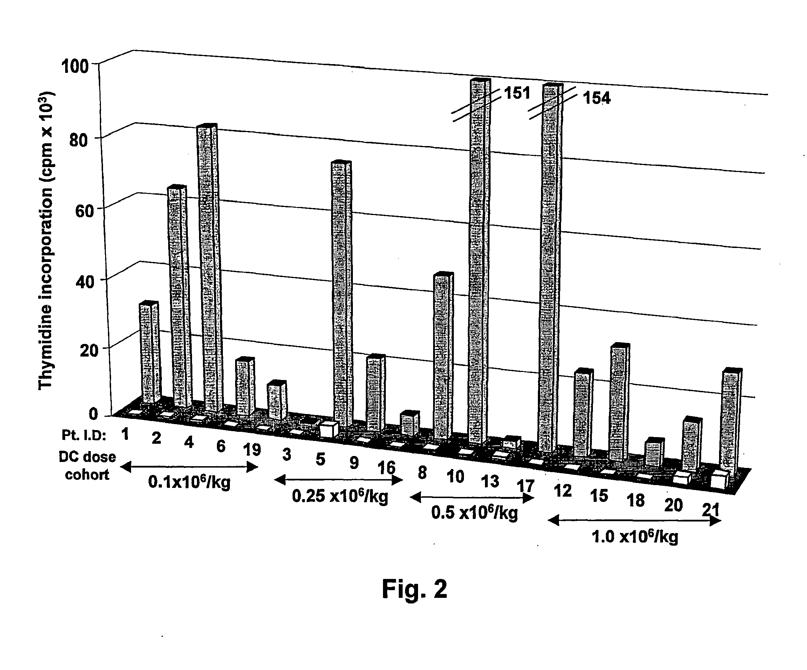

Fig. 2 depicts responses to control antigens: KLH dependent proliferative responses. Pre-/ post-immunization peripheral blood mononuclear cells (PBMCs) are cultured with 10 µg/ml KLH for 5 days. KLH-specific T cell proliferation is determined based on ³H TdR incorporation. Pre-immunization=white bars. Post-immunization=gray bars. KLH=keyhole limpet hemocyanin.

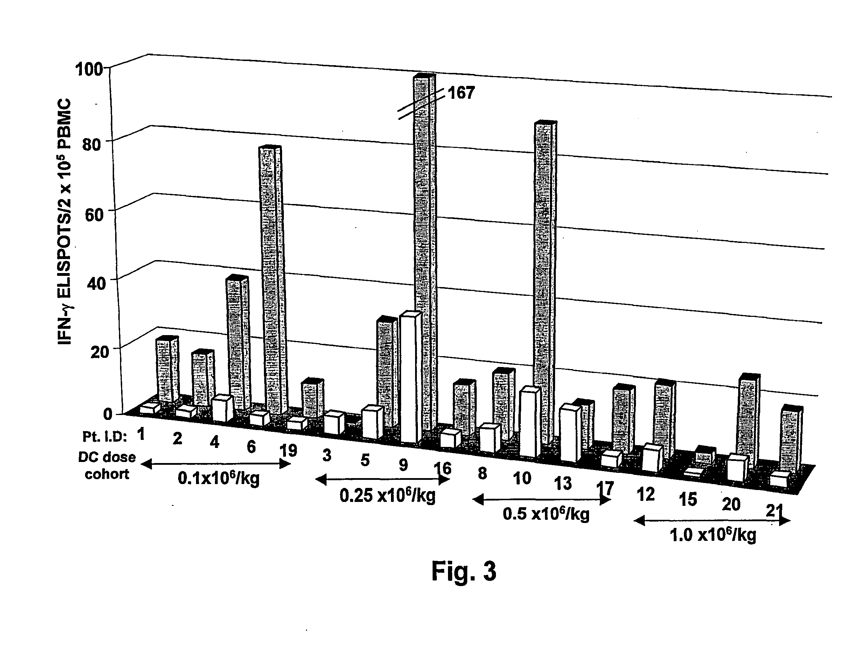

Fig. 3 depicts responses to control antigens: flu-MP peptide. PBMCs obtained at baseline and after 4 DC vaccinations are cultured overnight with 10 µM flu-MP peptide. Antigen specific IFN-γ secreting cells are quantified using an ELISPOT assay, and expressed as number of IFN-γ ELISPOTs / 2 x 10⁵ PBMC. The numbers obtained in control wells are subtracted (median 1 spot/2 x 10⁵ PBMC, range 0-8). Baseline=white bars. After 4 DC vaccinations=gray bars.

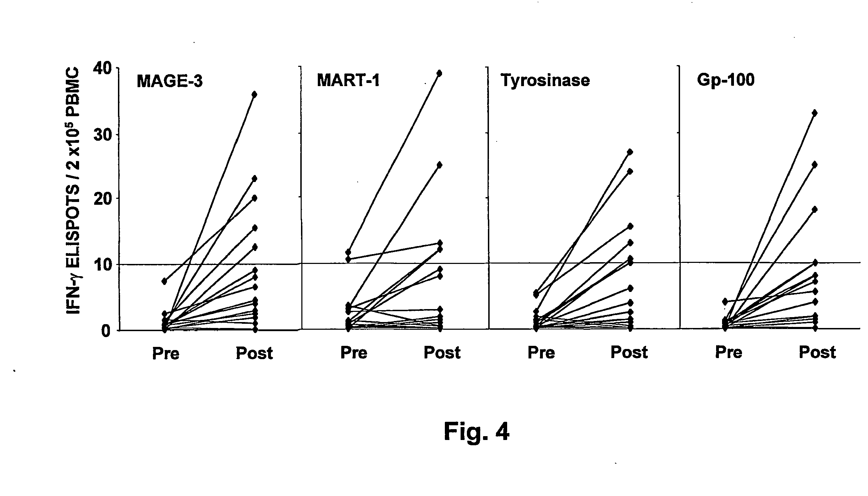

Fig. 4 depicts melanoma antigens-specific responses: circulating melanoma-specific effector cells detected in a short-time (overnight) assay. PBMC obtained at baseline and after 4 DC vaccinations are cultured overnight with each of the four melanoma peptides used for vaccination (10 µM). The specific T cell response in each of the evaluated patients is expressed as the number of IFN-γ ELISPOTS/2 x 10⁵ PBMC. From left to right: MAGE-3, MelanA/MART-1, Tyrosinase, and gp100. The values obtained with control gag peptide or in the wells with no peptide are subtracted (on average 1 spot/2 x 10⁵ PBMC, range 0-8).

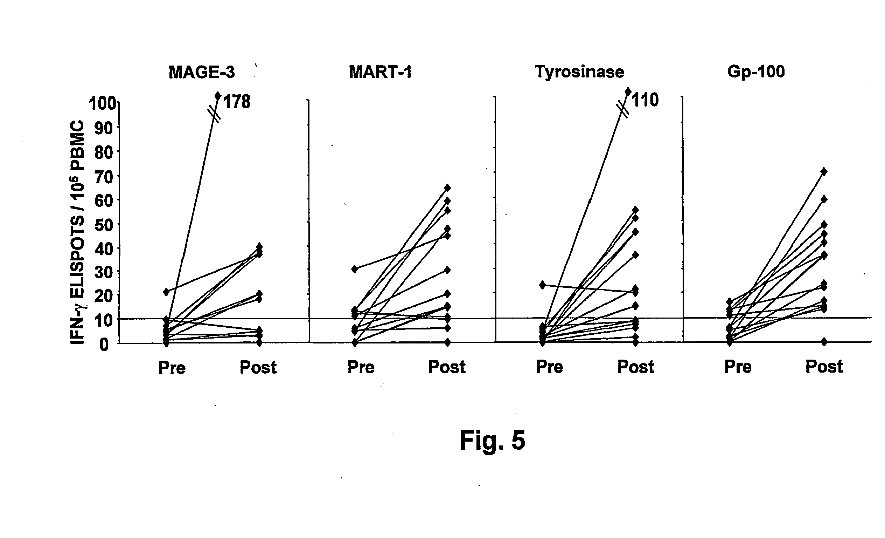

Fig. 5 depicts melanoma antigens-specific responses: expansion of melanoma-antigens specific memory effector cells (recall assay). PBMC obtained at baseline and after 4 DC vaccinations were cultured for 7 days with mature melanoma-peptide pulsed autologous monocyte-derived DC. The T cells were harvested, and peptide induced IFN-γ release was measured in the overnight culture with melanoma-peptide pulsed T2 cells. The response in each of the evaluated patients is expressed as the number of IFN-γ ELISPOTS/1 x 10⁵ PBMC. The values obtained with unpulsed T2 cells are subtracted; MAGE-3: median number of spots pre-/post-vaccine 1/7, range 0-25/0-23; MART-1: median number of spots pre-/post-vaccine 5/3, range 0-37/0-33; Tyrosinase: median number of spots pre-/post-vaccine 2/4, range 0-19/0-39; gp100: median number of spots pre-/post-vaccine 2/9, range 0-25/0-33.

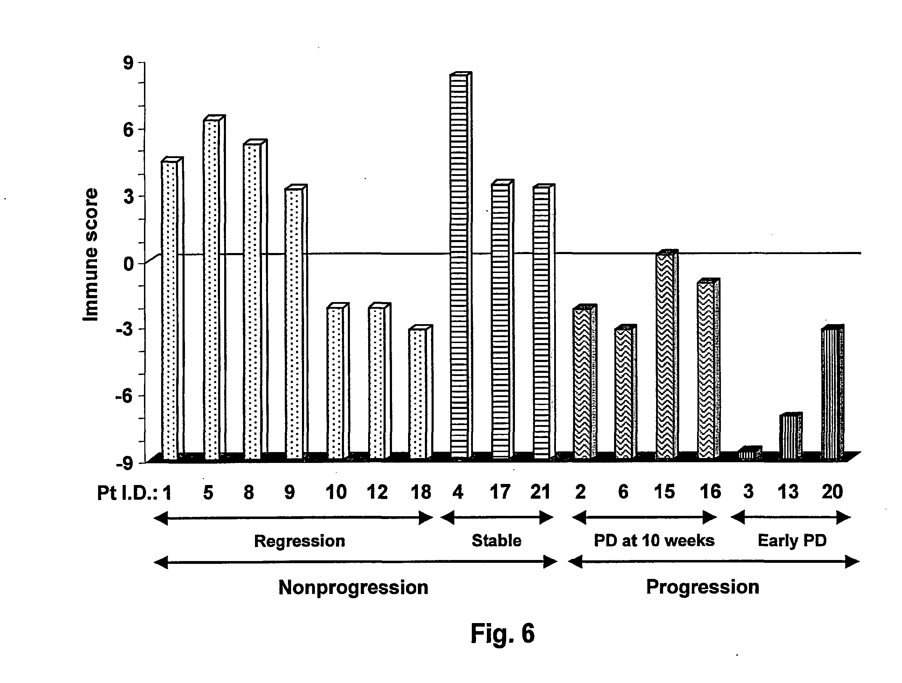

Fig. 6 depicts correlation of immune response and clinical outcome. Immune responses from both direct and recall assays for all 4 melanoma antigens were ranked and integrated into a tumor immunity score (from -8 to +8). Data are plotted as tumor immunity score (y axis) versus clinical response (x axis). Each number indicates the patient ID. Four groups of patients are depicted: patients with regression of one or more lesions post-vaccine (dotted bars), patients with stable disease (horizontal line bars), patients whose disease progressed at 10 weeks (PD, zigzag bars), and patients who experienced early progression (before evaluation at 10 weeks, early PD, vertical line bars).

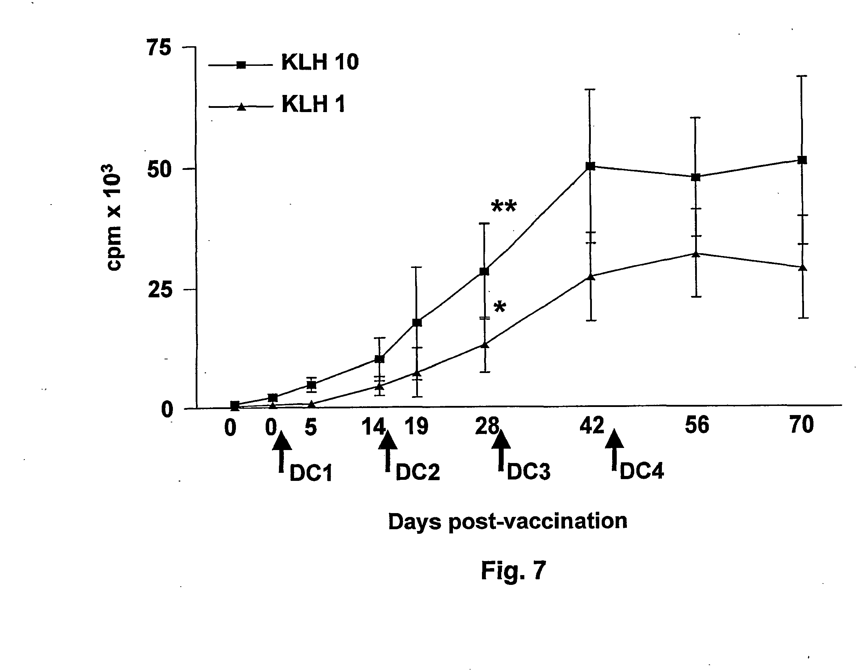

Fig. 7 depicts kinetics of KLH-specific responses. Peripheral blood mononuclear cells (PBMCs) collected pre-immunization (Day 0) and at different time points post-immunization (indicated on ordinate axis) are cultured with 10 µg/ml or 1 µg/ml KLH for 5 days. KLH-specific T cell proliferation is determined based on ³H TdR incorporation (vertical axis). * Significance in comparison of cpm values at the indicated time point over baseline (Day 0) (t-test) (* = p < 0.05; ** = p < 0.005).

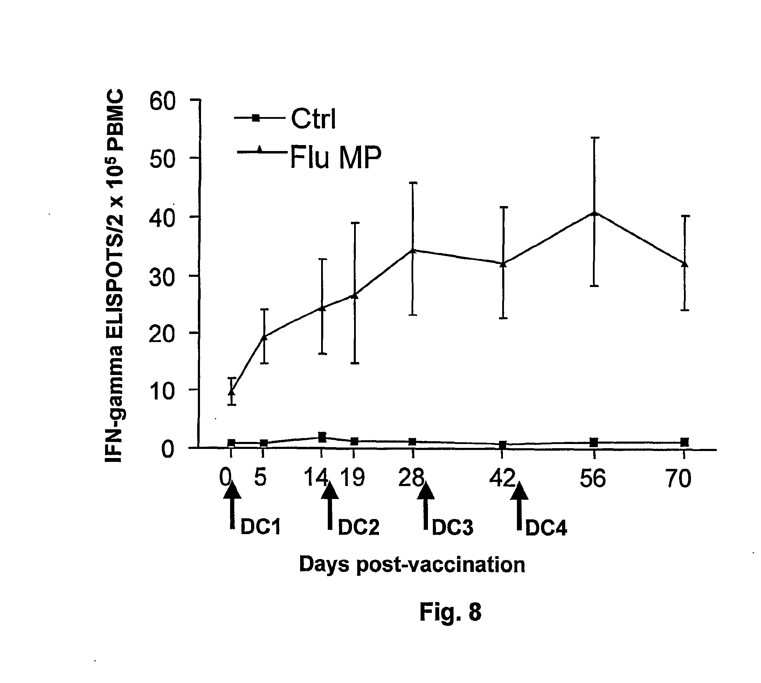

Fig. 8 depicts kinetics of Flu-MP-specific responses. PBMCs obtained at baseline and after indicated time points after DC vaccinations (ordinate axis) are cultured overnight with 10 µM flu-MP peptide or with a control peptide (hiv-gag). Antigen specific IFN-γ secreting cells are quantified using an ELISPOT assay, and expressed as number of IFN-γ ELISPOTs / 2 x 10⁵ PBMC.

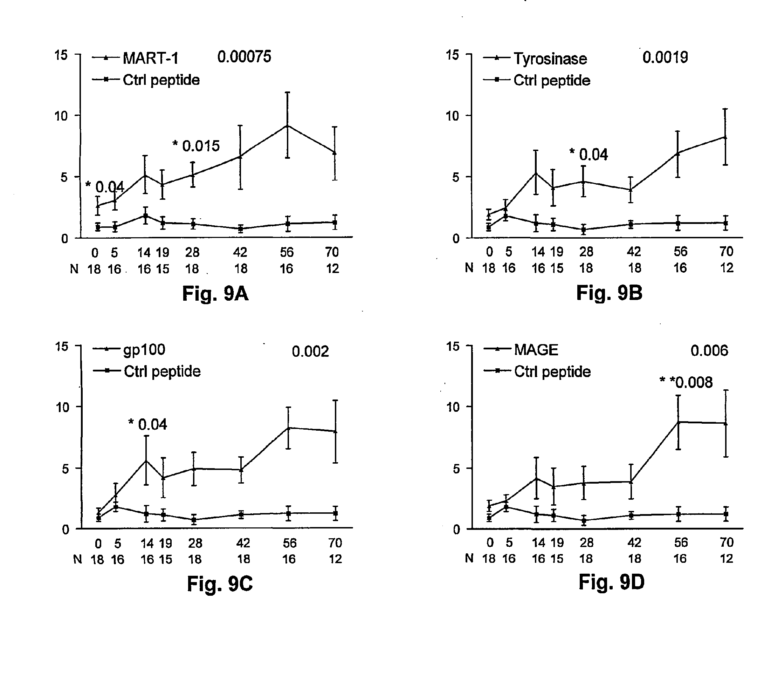

Fig. 9A-9D depict kinetics of melanoma-specific responses. PBMC obtained at baseline and after indicated days post-DC vaccinations (x axis) were cultured overnight with each of the four melanoma peptides used for vaccination (10 µM): MART-1 in Fig. 9A; Tyrosinase in Fig. 9B; gp100 in Fig. 9C; and MAGE in Fig. 9D. The specific T cell response in each of the evaluated patients is expressed as the number of IFN-γ ELISPOTS/2 x 10⁵ PBMC (y axis). The values obtained with control gag peptide or in the wells with no peptide are shown as control. N=number of patients whose samples were available for evaluation at the indicated time point, * Significance in comparison of ELISPOTS at the indicated time point over baseline (Day 0) (t-test). P values given in the upper right indicate significance in comparison of ELISPOTS for each peptide over control.

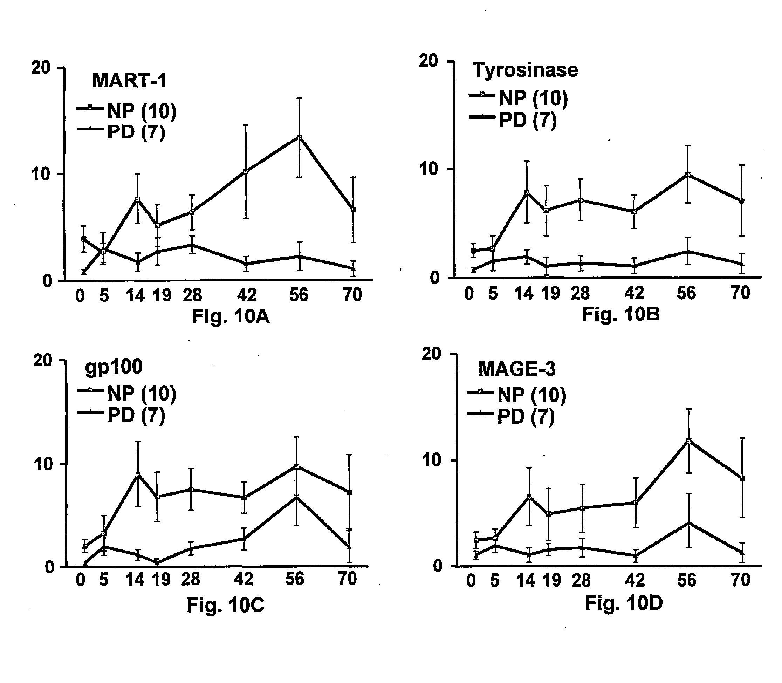

Fig. 10A-10D depict non-progressive patients mount higher TAA-specific responses in the blood than patients with tumor progression. PBMC obtained at baseline and after indicated days post-DC vaccinations (x axis) are cultured overnight with each of the four melanoma peptides used for vaccination (10 µM): MART-1 in Fig. 10A; Tyrosinase in Fig. 10B; gp100 in Fig. 10C; and MAGE in Fig. 10D. The specific T cell response in each of the evaluated patients is expressed as the number of IFN-γ ELISPOTS/2 x 10⁵ PBMC (y axis).

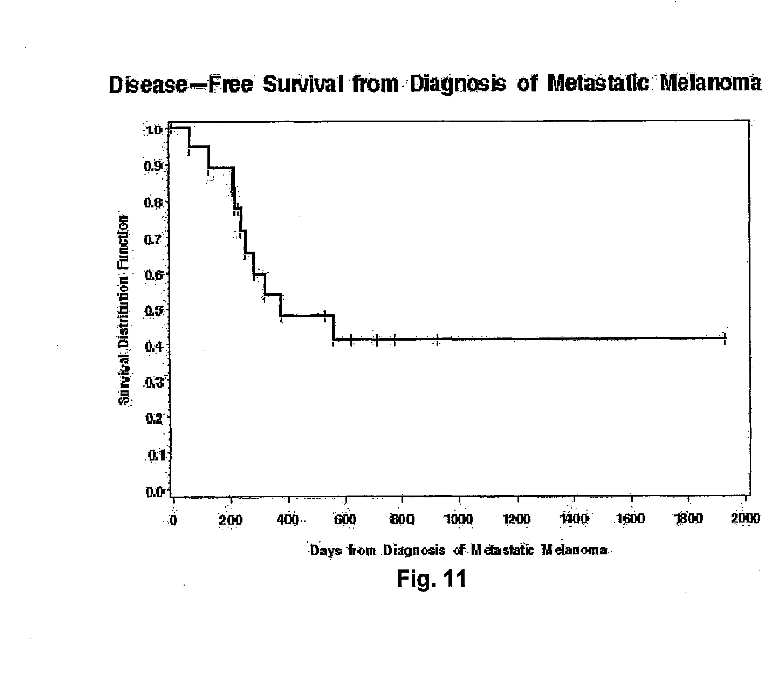

Fig. 11 depicts a survival curve for all patients enrolled in the study showing disease-free survival from the diagnosis of metastatic melanoma.

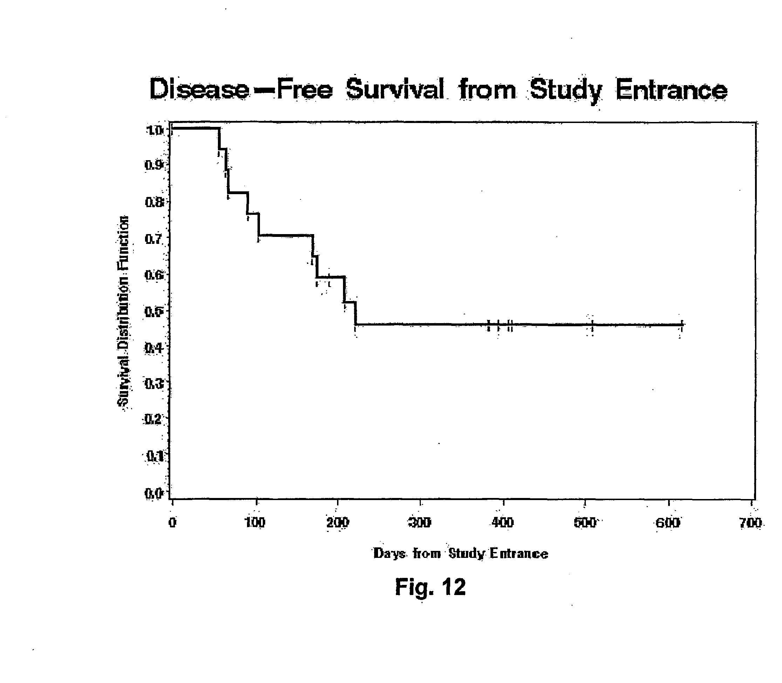

Fig. 12 depicts a survival curve for all patients enrolled in the study showing disease-free survival from the study entrance.

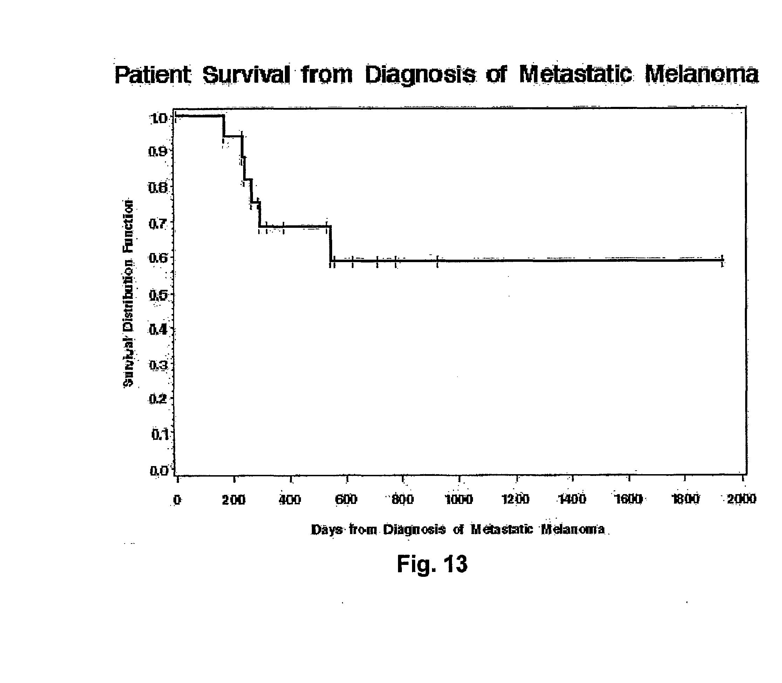

Fig. 13 depicts a survival curve for all patients enrolled in the study showing patient survival from the diagnosis of metastatic melanoma.

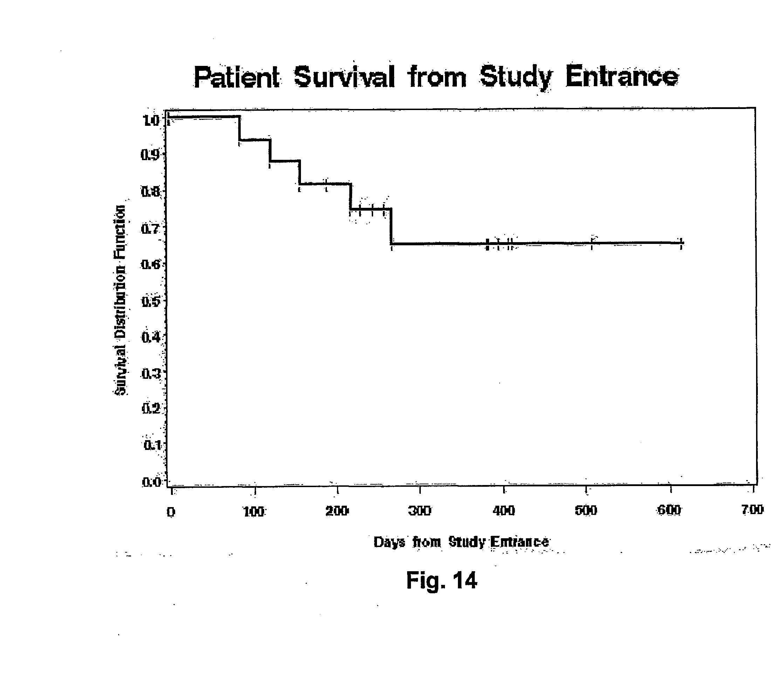

Fig. 14 depicts a survival curve for all patients enrolled in the study showing patient survival from the study entrance.

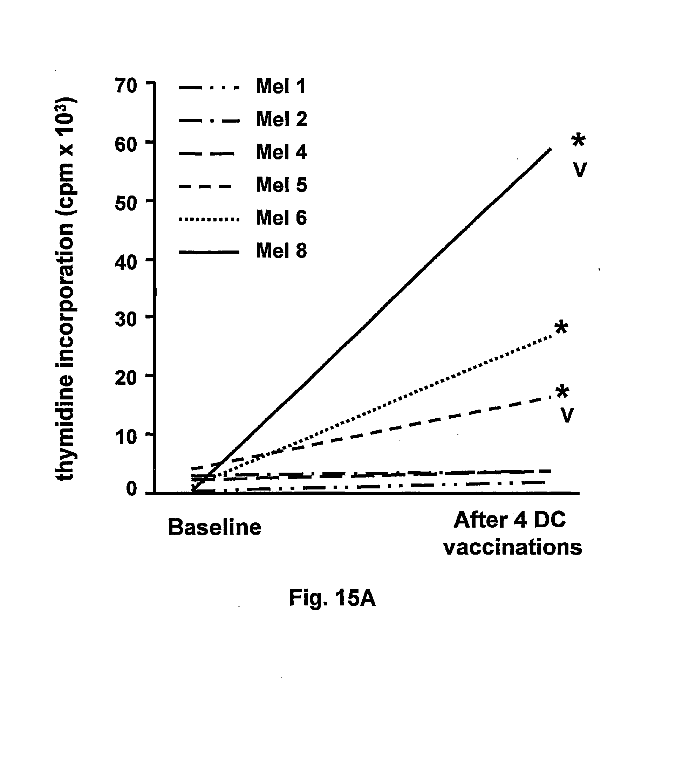

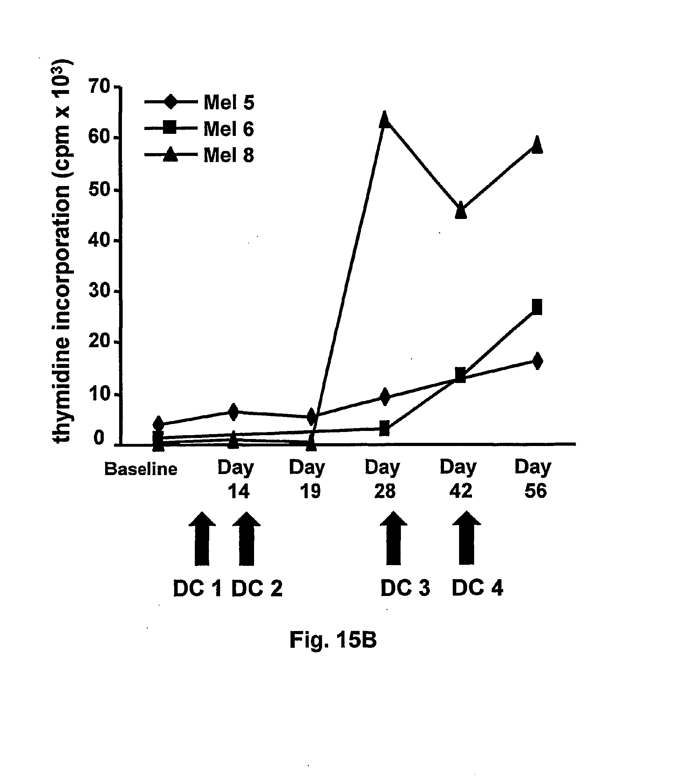

Fig. 15A and 15B depict the presence of spontaneous proliferation in some of the patients, wherein DC vaccination elicits in vitro "autoreactivity" in patients with metastatic melanoma. PBMC from post-DC immunization blood samples, cultured without adding exogenous antigens, proliferate; and in two patients, this is correlated with the presence of progressive vitiligo (Mel 5 and Mel 8). Fig. 15A shows thymidine incorporation (cpm x 10³) at baseline and at 2-4 weeks after the 4^{th} DC vaccine. Fig. 15B shows that spontaneous proliferation can manifest itself already after two DC vaccines.

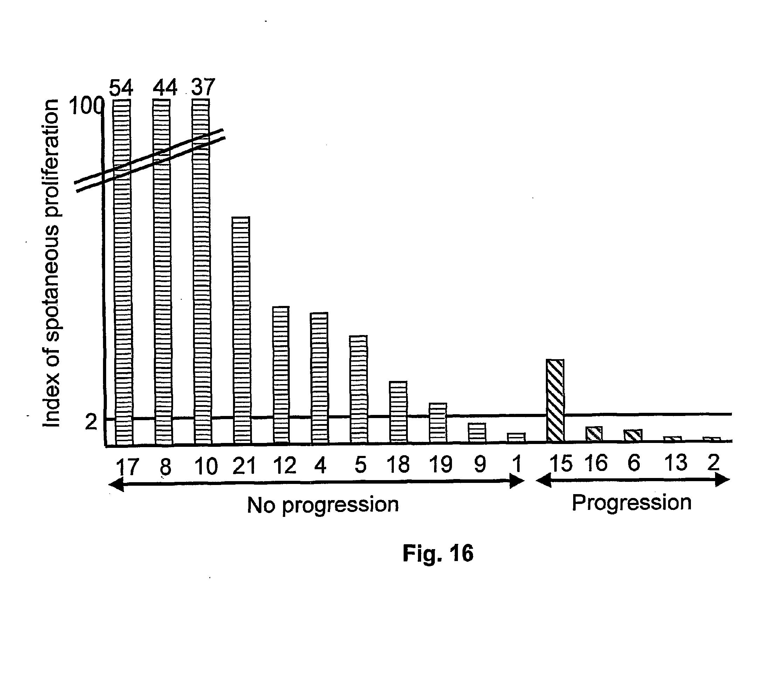

Fig. 16 depicts a correlation between the level of spontaneous proliferation in post-vaccination blood samples and the clinical responses. The index of spontaneous proliferation (vertical axis) is calculated as the ratio of cpm values from 5 days cultures of PBMC post-vaccination over pre-vaccination. Ordinate axis depicts patient number and clinical status at 10 weeks.

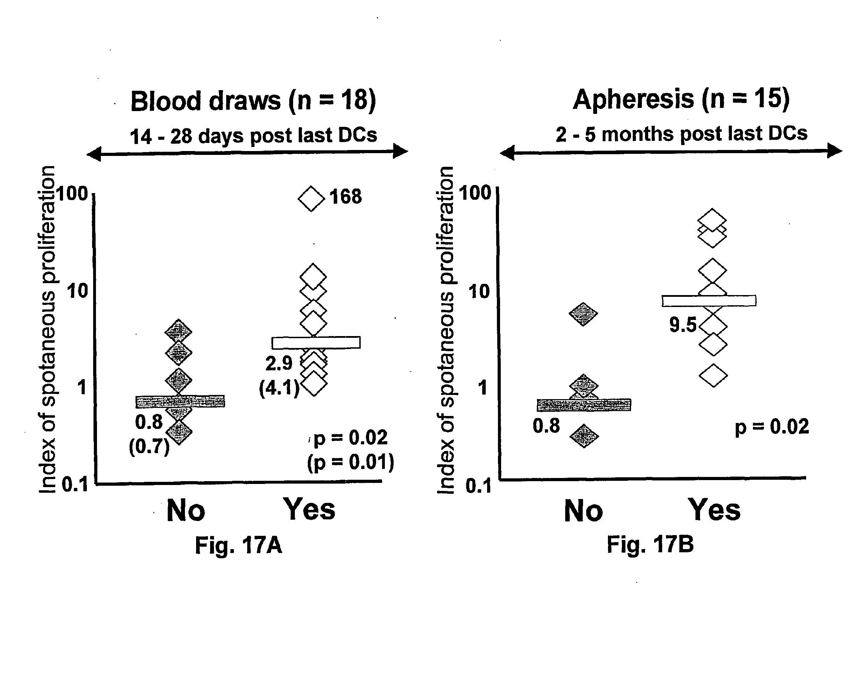

Fig. 17A and 17B depict a correlation between the level of spontaneous proliferation in post-vaccination blood samples and the clinical responses. The index of spontaneous proliferation (vertical axis) is calculated as the ratio of cpm values from 5 days cultures of PBMC post-vaccination over pre-vaccination. Fig. 17A displays analysis at 14-28 days post last DC vaccination (assays were done blind in one lab) and Fig. 17B shows analysis at 2-5 months post vaccinations (assays were done blind in another lab). P values indicate level of significance in t-test. Values in brackets in Fig. 17A reflect values obtained with the samples from the same 15 patients shown in Fig. 17B, otherwise values in Fig. 17A reflect the analysis of 18 patients.

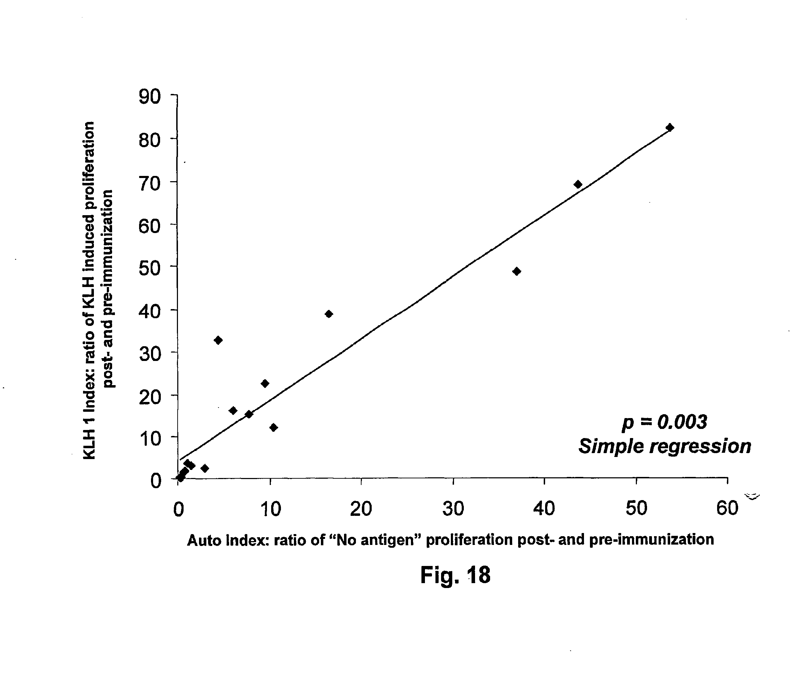

Fig. 18 depicts correlation between the level of spontaneous responses and antigen-specific responses induced by DC vaccination, wherein antigen-specific responses induced by DC vaccine are correlated to "autoreactive" responses. PBMC from pre- and post-DC immunization samples are cultured with KLH or without adding exogenous antigens. Proliferation is measured by thymidine incorporation at Day 5. The response index is calculated as a ratio of cpm (from double triplicates) obtained from samples post and pre-immunization.

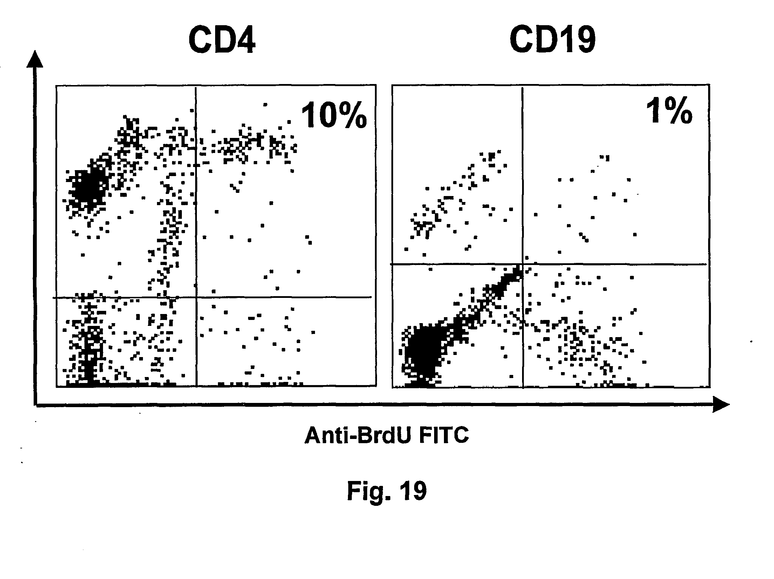

Fig. 19 depicts the spontaneously proliferating cells are CD4 T cells and only very few CD19+ B cells. Post-vaccination PBMC are cultured for 5 days. The cultures are spiked with BrdU for the last 16 hours, and BrdU incorporation by CD4 positive T cells or by CD19+ B cells is revealed by staining with anti-BrdU FITC-conjugated antibody and flow cytometry.

### DETAILED DESCRIPTION OF INVENTION

This invention is directed to method of treating malignancies through induction of immune responses. According to the present invention, the elicitation of blood immune responses, including vaccine-specific ones, relates to clinical response in the treatment of malignancies. The evaluation of vaccine-induced immune responses in blood provides a predictive factor for treatment efficacy.

An important aspect of the present invention is a novel and unifying concept of a multi-pronged approach to treating malignancies through induction of immune responses. This novel approach incorporates at least two of the following four key factors: 1) an appropriate mode or vector for vaccination, e.g., using dendritic cells, 2) the use of multiple tumor-associated antigens, 3) the use of control antigens, and 4) comprehensive evaluation of the elicited immune responses using multiple but simple assays.

In the present invention, vaccines are administered to patients diagnosed with malignancies to effect a reduction in disease progression. Furthermore, vaccines of the present invention can be administered prophylactically to reduce the probability of an individual developing a malignancy.

In the method of the present invention, a patient with tumor disease, including but not limited to, metastatic melanoma is injected subcutaneously with an appropriate vector for vaccination, including but not limited to autologous DCs derived from CD34+ progenitors. The vector for vaccination contains multiple tumor antigens, for example peptides derived from multiple melanoma antigens (e.g., MelanA/MART-1, tyrosinase, MAGE-3, and gp100). The vector for vaccination can also include other antigens including but not limited to control antigens, for example influenza matrix peptide (Flu-MP); or keyhole limpet hemocyanin (KLH). In one embodiment of the method of present invention, a single vector for vaccination contains multiple antigens including but not limited to tumor antigens, and optionally control antigens. In another embodiment of present invention, antigens can be delivered by administration of multiple vectors each containing a single antigen. A similar protocol would be followed for prophylactic treatment.

The vector for vaccination used in the present invention includes but is not limited to dendritic cells generated from CD34 hematopoietic progenitors, for example, including but not limited to Langerhans cells and interstitial dendritic cells. The vector used for vaccination may be composed of dendritic cells or other antigen-presenting cells, or the vaccine may target dendritic cells or other antigen-presenting cells when delivered to patient.

An important aspect of the present invention is a novel method to generate dendritic cells to be used as vector for vaccination under the conditions that permit injection of these dendritic cells into a patient. According to the method of the present invention, dendritic cells are generated from CD34 hematopoietic progenitors by culturing these in a tissue culture medium that is not supplemented by any foreign serum components such as fetal bovine serum. This tissue culture medium does not contain any serum components or is supplemented with autologous serum. In a method of present invention, autologous serum is not heat inactivated and can be used at 5% or 10% (v/v). In the method of present invention, CD34 hematopoietic progenitors are cultured preferably for 9 days. All cells that form in tissue cultures of the present invention are considered as a vector for vaccination.

The vaccine used in the present invention may be any composition which induces blood immune responses. Preferably, the vaccine is composed of several antigens including several tumor antigens. Exemplary tumor antigens include those representing the four melanoma antigens: Melan A/MART-1, tyrosinase, gp100, and MAGE-3. Other exemplary antigens include control antigens, for example, viral antigens, flu matrix peptide, or protein antigens (e.g., KLH).

In the method of the present invention, immune responses against control antigens, including but not limited to KLH and flu matrix peptide, indicate patient's immune competence and are predictive of the immune responses against tumor antigens. The lack of responses against control antigens, including but not limited to KLH and flu matrix peptide, is predictive of the lack of responses to tumor antigens and of early disease progression.

In the method of the present invention, the vaccine leads to induction of immune responses against unidentified antigens, for example, "spontaneous" proliferation. These unidentified antigens may be present in the vaccine preparation or induced in the course of vaccination. According to the present invention, immune responses against such antigens indicate a patient's immune competence and are predictive of the patient's immune responses against tumor antigens. The immune responses against such antigens, including but not limited to unidentified antigens present either in the vaccine preparation or induced in the course of vaccination, are also predictive of the clinical response.

The route of vaccine administration in the present invention includes but is not limited to subcutaneous, intracutaneous or intradermal injection. In the present invention, patients should be vaccinated for a life-time or until progression of malignancy. In the case of malignancy progression, the vaccine should be modified, including but not limited to the use of novel antigens. Similar protocol would be followed for prophylactic treatment.

In the present invention, the vaccine treatment consists of at least two phases including but not limited to induction phase and consolidation phase. In the induction phase, the vaccine is administered at shorter intervals, preferably but not limited to every other week. In the consolidation phase, the vaccine is administered at longer intervals, preferably but not limited to on a monthly basis. The consolidation phase may be followed by boost immunizations, preferably but not limited to every 3-6 months.

In the present invention, the comprehensive evaluation of elicited immunity against control antigens and tumor antigens can be determined by any means known in the art. For example, a PBMC proliferation assay can be used, wherein PBMCs obtained from the patient before and after immunization are cultured without adding exogenous antigen or in the presence of the antigen and T cell proliferation is subsequently determined. In another method, ELISPOT is used to determine the presence of functional T cells specific for tumor antigens, including but not limited to circulating effector T cells (overnight assay) and blood memory T cells (simple recall assay), which is predictive of early clinical outcome in a vaccination trial. Preferably, the elicited immune responses to melanoma antigens are determined at two levels: 1) circulating effector T cells in an overnight assay, and 2) memory T cells in a 1-week recall assay with single ex vivo T cell stimulation.

According to the method of the present invention, the frequency of vaccine administration can be individualized based on evaluating blood immune responses after the first vaccination, preferably at Day 5 and at Day 14. The presence of immune responses at such an early stage identifies patients that require less frequent vaccination in the induction phase, for example on a monthly basis. The absence of immune responses at this stage identifies patients that require more frequent vaccination in the induction phase, preferably every other week.

The evaluation of the overall tumor immunity score for a patient is associated with early clinical outcome. Overall immunologic effects of treatment are preferably assessed by one or both of the following methods: 1) comparing response profiles using marginal likelihood scores, and/or 2) classifying patients according to observed results, including but not limited to the presence or absence of the induction of spontaneous PBMC proliferation (i.e., without adding exogenous antigen); presence or absence of the induction of immune responses to control antigens; presence or absence of the induction of immune responses to melanoma antigens ("MelAgs") in any of the assays; and/or presence or absence of induction of immune responses to more than two melanoma antigens. The induction of spontaneous PBMC proliferation is predictive of early favorable clinical outcome in vaccination trial. Absence of spontaneous PBMC proliferation is predictive of early disease progression in the vaccination trial. The induction of immune response against at least two tumor antigens in any of the assays, including but not limited to circulating effector T cells (overnight assay) and blood memory T cells (simple recall assay), is predictive of early favorable clinical outcome in the vaccination trial. The presence of immune response against less than two tumor antigens in any of the assays, including but not limited to circulating effector T cells (overnight assay) and blood memory T cells (simple recall assay), is predictive of early disease progression in the vaccination trial.

### EXAMPLE 1: Comparison of Elicited Immune Responses to Clinical Response

To evaluate the method of the present invention, 18 HLA A*0201⁺ patients with metastatic melanoma were injected subcutaneously with autologous DCs derived from CD34+ progenitors, which included a distinct Langerhans cell component. DCs were pulsed with peptides derived from 4 melanoma antigens (MelanA/MART-1, tyrosinase, MAGE-3 and gp100), as well as influenza matrix peptide (Flu-MP) and keyhole limpet hemocyanin (KLH) as control antigens. The elicited immune responses were determined using a PBMC proliferation assay for KLH and ELISPOT assay for flu-MP and melanoma antigens (MelAgs). Melanoma-specific responses were evaluated at two levels: 1) circulating effector T cells in an overnight assay, and 2) memory T cells in a 1-week recall assay with single ex vivo T cell stimulation. Overall immunologic effects of treatment were assessed by two methods: 1) comparing response profiles using marginal likelihood scores, and 2) classifying patients according to observed results regarding the induction of responses to control antigens, a MelAg in any assay, and more than two MelAgs.

DCs induced an immune response to control antigens (KLH, Flu-MP) in 16 of 18 patients. An enhanced immune response to one or more melanoma antigens (MelAg) was seen in these same 16 patients, including 10 patients who responded to > 2 MelAg. We first used > 2-fold increase and > 10 antigen specific IFN-γ ELISPOTS in the post-vaccine assays as an indicator of immune response. Two patients (# 3 and #13) who failed to respond to either the control or melanoma antigens by any assay, experienced rapid tumor progression and could not complete the planned therapy. Of 17 patients with evaluable disease, 6 of 7 patients who responded to 0,1, or 2 melanoma antigens had progressive disease upon restaging 10 weeks after study entry. In contrast, tumor progression was seen in only 1 of 10 patients who responded to 3 or all 4 melanoma antigens (p=0.002, Fisher exact test). Regression of > 1 tumor metastases were observed in 7 of these patients.

In order to obtain overall assessment of anti-tumor immunity after the DC vaccine, we integrated data for absolute immune responses to all 4 antigens by both direct and recall assays into a tumor immunity score (from -8.5 to +8.5). Among the 17 patients with evaluable disease, tumor immunity was lower among the 7 patients with progressive disease (p=0.043). The tumor immunity score was associated to the clinical outcome. Six of 8 patients with a negative tumor immunity score had progressive disease. On the other hand, only 1 of the 9 patients with a positive tumor immunity score progressed. Therefore, both these analyses show that early clinical outcome after DC vaccination correlated to the elicited immune response.

### Materials and Methods

### Study Design and Eligibility Criteria:

The study schema is shown in Figure 1. Inclusion criteria were biopsy proven AJCC stage IV metastatic melanoma; age ≥ 18 years; Karnofsky performance status >80%; HLA-A*0201 phenotype; intradermal skin test positivity to mumps, histoplasmosis, or streptokinase antigen; normal blood CD4 and CD8 T-cell numbers by flow cytometry; and normal quantitative immunoglobulin levels. Exclusion criteria were: prior chemotherapy or biologicals < 4 weeks before trial entry; untreated CNS lesions; bulky hepatic metastatic lesions; pregnancy; or concurrent corticosteroid and/or immunosuppressive therapy. Patients with history of asthma, venous thrombosis, congestive heart failure, autoimmune disease, or active infections including viral hepatitis were also excluded. All patients gave a written informed consent and the study was approved by the Food and Drug Administration, the National Cancer Institute (NCI) and the Institutional Review Board. Patients received a 6-week outpatient vaccination course with antigen-loaded CD34 DC given subcutaneously (s.c.) every 14 days for a total of four vaccinations. DCs were administered in a dose escalation design at the dose level per cohort of 0.1, 0.25, 0.5, and 1 x 10⁶ DC/kg/injection.

### Preparation and administration of the DC vaccine:

### Harvest of DC progenitors:

The patients received recombinant G-CSF (Neupogen; Amgen, Thousand Oaks, CA) 10 µg/kg/day s.c. for 5 days, for peripheral blood stem cell mobilization, and then underwent leukapheresis for two consecutive days to collect mobilized CD34+HPC. The cells were processed using the CEPRATE SC stem cell concentration system (CellPro Inc., Seattle, WA) to obtain an enriched population of CD34⁺ HPC (purity 62 ± 17%; recovery 158 ± 133 x 10⁶) which were then cryopreserved.

### Preparation of DC vaccine:

All procedures were performed according to Good Laboratory Practice (GLP) standards. CD34-DCs were generated from CD34⁺ HPC by culture at a concentration of 0.5 x 10⁶/ml culture medium (X-VIVO-15, BioWhittaker, Walkersville, MD) supplemented with autologous serum, 10⁻⁵ M 2-β-mercaptoethanol and 1% L-glutamine. The following human recombinant cytokines, approved for clinical use, were used: GM-CSF (50 ng/ml, Immunex Corp., Seattle, WA), Flt3-L (100 ng/ml, Immunex Corp.) and TNF (10 ng/ml, CellPro, Inc., Seattle, WA). Cultures were conducted in a humidified incubator at 37°C and 5% CO₂ with a separate incubator being assigned to each patient. On Day 8 of culture, 20% of cells were pulsed overnight with KLH (2 µg/ml, Intracell Corp., Rockville, MD) and with HLA-A*0201 restricted flu-matrix peptide (Flu-MP) GILGFVFTL₅₈₋₆₆ (2.5 µg/ml). Remaining cells were pulsed overnight with KLH (2 µg/ml) and with 4 HLA-A201 restricted peptides (2.5 µg/ml) derived from melanoma antigens (MelanA/MART-1₂₇₋₃₅: AAGIGILTV, gp100g_{209-2M}: IMDQVPFSV, Tyrosnase₃₆₈₋₃₇₆: YMDGTMSQV, MAGE-3₂₇₁₋₂₇₉: FLWGPRALV). All peptides were GMP quality and were either obtained from the NCI (MelanA/MART-1, gp100 and tyrosinase) or purchased (Flu-MP and MAGE-3, MultiPeptide Systems, San Diego, CA). After overnight loading with antigens, CD34-DCs were washed three times with sterile saline, counted and resuspended in 10 m1 of sterile saline containing melanoma peptides (1 µg/ml). After two hours incubation at 22°C, the cells were centrifuged, and resuspended in 9 ml of sterile saline for injection. Vaccine release criteria included: 1) negative bacterial culture 48 hours prior to DC injection, 2) negative Gram-staining after antigen pulsing, 3) dendritic cell morphology on Giemsa stained cytospins performed two hours before DC administration, 4) cell viability > 80%, and 5) a minimum of 20% DC (CD1a+ and CD14+) in cell preparation as determined by phenotypic analysis. Further quality testing of each DC batch included 1) reactivity with a panel of monoclonal antibodies; and 2) determination of their stimulatory capacity in mixed lymphocyte reactions.

### Administration of Vaccine:

Vaccination was administered intracutaneously in three injection sites (both thighs / upper arm). Limbs where draining lymph nodes had been surgically removed and/or irradiated, were not injected. DCs were injected using a long spinal-cord needle and spread over a 6-8 cm distance.

### Clinical Monitoring

Adverse events were graded according to the NCI Common Toxicity Criteria. All patients underwent assessment of tumor status at baseline and 4 weeks after the fourth DC vaccination (10 weeks from trial entry). Disease progression was defined as > 25% increase in target lesions and/or the appearance of new lesions.

### Immunologic Monitoring

PBMC samples were harvested and frozen from at least 2 time points before vaccination, as well as 5 and/or 14 days after each vaccination, and 14 or 28 days after the 4^{th} vaccination. Pre- and post-immunization PBMCs were frozen in aliquots, coded, thawed and assayed together in a blinded fashion.

### Antigen specific proliferation

PBMCs (10⁵ cells/well) were cultured in triplicate wells in the absence or presence of graded doses of KLH at 1-10 µg/ml, and as a positive control, staphylococcal enterotoxin A (SEA). Assays were pulsed overnight with ³H thymidine on Day 3 (SEA) or 5 (KLH) of culture and harvested 16 hours later.

### ELISPOT assay for IFN-γ release from single antigen specific T cells

ELISPOT assay for the detection of antigen specific IFN-γ producing T cells was performed as previously described (Dhodapkar, et al.1999. "Rapid generation of broad T-cell immunity in humans after a single injection of mature dendritic cells," J Clin Invest 104:173-180; and Dhodapkar, et al. 2000. "Mature dendritic cells boost functionally superior CD8(+) T-cell in humans without foreign helper epitopes," J Clin Invest 105:R9-R14). Briefly, PBMCs (2 x 10⁵ cells/well) were added to plates precoated with 10 µg/ml of a primary anti-IFN-γ mab (Mabtech, Stockholm) in the presence or absence of 10 µg/ml peptide antigens. The antigens were the same HLA A*0201 restricted peptides (4 melanoma peptides and Flu-MP) used in the DC vaccine. HLA A*0201 restricted gag peptide was used as a negative control, and SEA as a positive control for T cell function. For some experiments, depending on the cell yield, influenza virus infected PBMCs (MOI 2) were utilized as APCs. Antigen specific SFCs were calculated after subtracting the background with control peptide. Immune responses were scored as positive if the post-immunization measurements for antigen specific spot forming cells were > 2-fold higher than the baseline and > 10 SFC/2 x 10⁵ cells.

### Antigen-specific recall T cell responses

To evaluate the ability of antigen specific T cells to proliferate and differentiate in culture, pre-and post-immunization PBMC were thawed together and co-cultured (2 x 10⁵ cells / well) for 7 days with autologous mature DCs (PBMC:DC ratio 30:1) pulsed with 1 µg/ml peptides. After 7 days, cells were transferred to an ELISPOT plate and cultured overnight with (T: APC ratio 20:1) irradiated (3000 rads) T2 cells with or without specific antigen. Antigen-specific SFCs were calculated after subtracting the background with unpulsed T2 cells.

### Delayed Type Hypersensitivity (DTH) reactions.

To test for hypersensitivity reactions, 10⁵ CD34-DCs pulsed separately with each antigen were injected i.d. on the patient's back and induration at the injection site was measured at 48 hours.

### Statistical analysis

The sign test for discretized data was used to demonstrate the presence of specific immune response to KLH and Flu-MP (Wittkowski, K. M. 1998. "Versions of the sign test in the presence of ties," Biometrics 54:789-791). As the role of different melanoma antigens with regard to protective immunity is not known, we integrated post-vaccination responses to all 4 melanoma antigens, as measured by both direct and recall assays, into an immunity score using a non-parametric method based on the marginal likelihood approach (Kalbfleisch, J. D. and Prentice, R. L. 1973. "Marginal likelihoods based on Cox's regression and life model," Biometrika 60:267-278; and Wittkowski, K. M. 1988. "Friedman-type statistics and consistent multiple comparisons for unbalanced designs," J Am Stat Assoc 83:1163-1170). To score n individuals according to their immune response profiles, one computes all rankings (permutations of numbers 1...n) that are compatible with all pairwise orderings. As a result, a person with a higher immune response is assigned a higher rank. An immune response is considered higher, if it is at least as high for each of the 8 variables and higher for at least one variable. A patient's immunity score is the average of the corresponding ranks among the compatible rankings minus the expected score. All immunized patients were included in the analysis in an "intent to treat" approach.

The results of this study are summarized below.

### Patient Characteristics

Table I gives patient characteristics and disease status on entry and post-DC vaccine. Chemotherapy included DTIC, Cisplatinum and Velban; all CNS lesions were surgically removed or irradiated before trial entry. LN: lymph nodes(s), CE: clinical examination, PD: progressive disease. Patients #4 and #8 had mild pre-existing vitiligo that progressed during DC vaccination.
Eighteen HLA-A201⁺ patients with metastatic melanoma were injected with CD34-DCs (Fig. 1 and Table I). Four patients (Patient #1, #9, #12 and #13) had CNS involvement treated by surgery and radiation prior to entry on the trial, 4 patients (Patient #2, #4, #5 and #16) had received prior chemotherapy, and 5 patients (Patient #2, #4, #6, #9 and #15) had received prior biological therapy without a clinical or immune response (Table I). Three patients (Patient #3, #13 and #20) progressed before completing the trial.

### DC vaccine

Fresh DCs were generated from G-CSF mobilized blood CD34⁺HPC for each vaccination. Frozen/thawed CD34⁺HPC cultured for 9 days with GM-CSF, TNF-α and FLT3 ligand yielded MHC class I⁺, HLA-DR⁺, CD80⁺, CD86^{low} and CD83^{low} DCs (data not shown). The DCs included CD1a⁺CD14⁻ Langerhans cells (LC) as well as CD1a^{±}CD14⁺ interstitial DC precursors (intDC). The LC phenotype was confirmed by confocal microscopy revealing Langerin staining in CD1a⁺ DCs (data not shown) (Valladeau, et al. 2000. "Langerin, a novel C-type lectin specific to Langerhans cells, is an endocytic receptor that induces the formation of Birbeck granules," Immunity 12:71-81). The mean proportion of CD1a⁺CD14⁻ cells was 9±13% (range: 4-17%, median=9%) and that of CD1a^{±}CD14⁺ cells was 32 ^{±} 9% (range: 19-52%, median=30%).

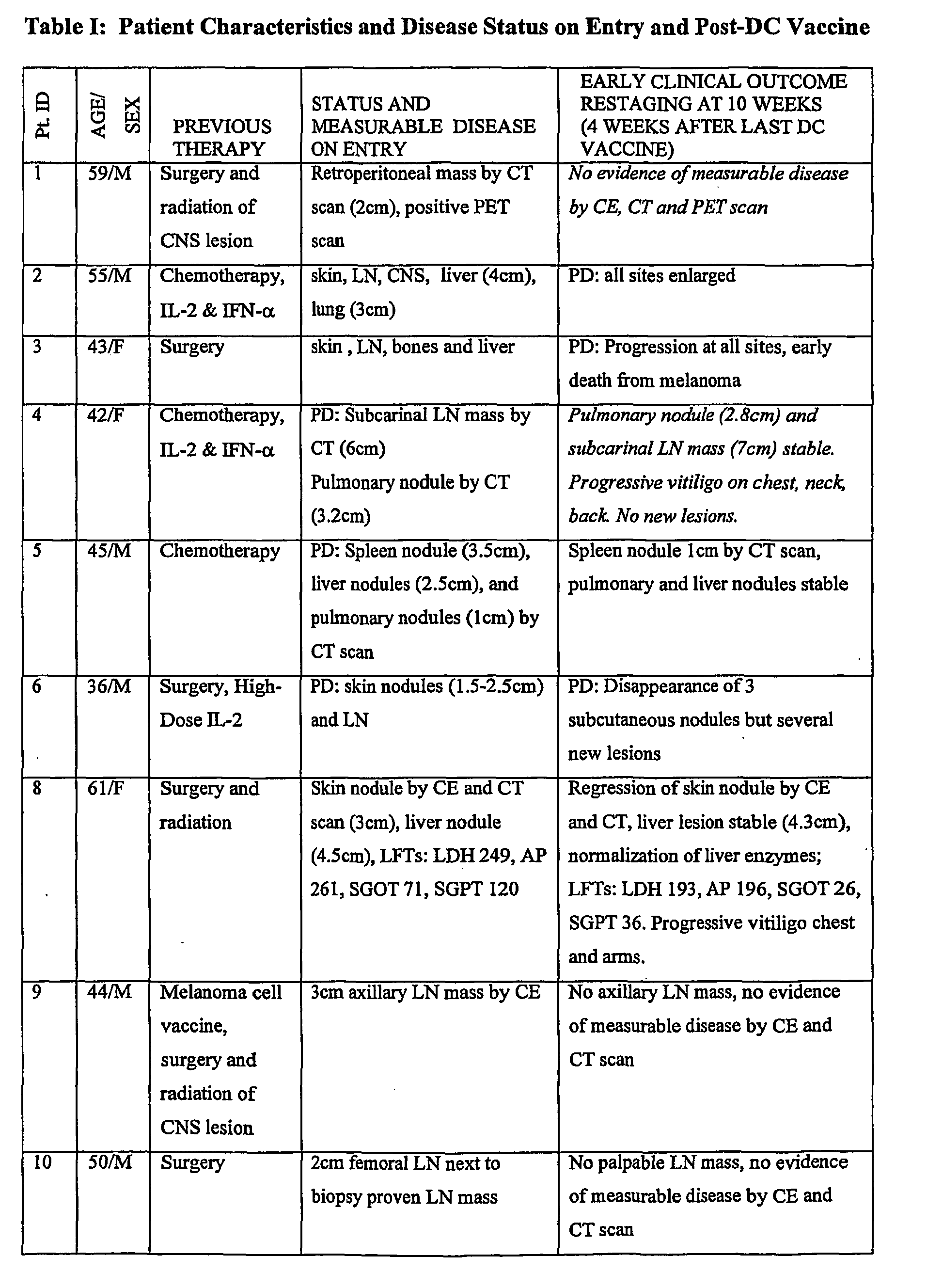

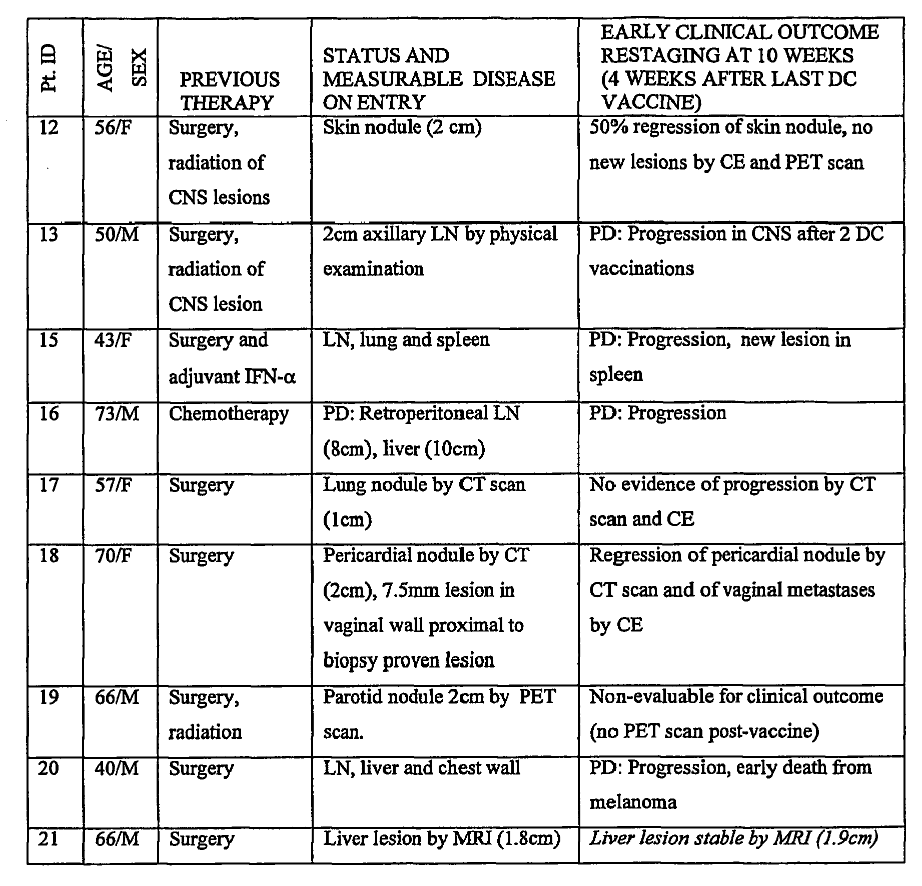

### Responses to control antigens

DC vaccination primed KLH specific immune responses in 16 of 18 patients (all except Patient #3 and #13) (*p=0.00007* in the exact sign test, Fig. 2). There was no indication that higher DC doses induced greater KLH specific proliferation. Sixteen patients (all except Patient #3 and #20) were evaluated for DTH after DC vaccination. Thirteen of these patients (all except Patient #1 and #19) developed DTH to KLH (median 10 mm, range: 6-47 mm) (data not shown).

Table II gives the number of peptide antigen specific IFN-γ ELISPOTS in pre-/post-vaccination PBMCs. Data shown are for circulating effectors (direct; SFC / 2 x 10⁵ cells) and after ex vivo expansion with antigen-pulsed DCs (recall; SFC/10⁵ cells). Data are shown as pre-/post-4^{th} vaccine, except for Patients #3, #13 and #20, all of whom experienced early progression. Patients #3 and #20 completed four vaccines, but the blood samples after the 4^{th} vaccine were not available (responses after three vaccines are shown). Patient #13 had CNS progression after two vaccines, was treated with CNS irradiation and Decadron, and completed the following vaccines outside the protocol (responses after two vaccines are shown). ND represents samples not assayed due to low cell yields.

Of the 17 patients injected with Flu-MP-pulsed DCs (all except Patient #18 with a history of allergy to flu vaccine), enhancement of Flu-MP specific memory T cell responses by at least one assay were observed in 15 patients (all except Patient #3 and #13) (Table II and Fig. 3). The elicited T cells also recognized the naturally processed antigen from flu infected PBMCs (data not shown).

The finding that all except 2 patients responded to both KLH and Flu-MP pulsed DCs indicates that patients with metastatic melanoma enrolled in our study were immunocompetent.

### In vivo expansion of melanoma-specific blood CD8 T cells

### Responses in uncultured T cells

Except for Patient # 8, only few melanoma antigen-specific IFN-γ producing cells were detected in baseline blood samples (Table II and Fig. 4). The response was considered as enhanced if there was > 2-fold increase and a minimum of 10 MelAg specific ELISPOTs (after subtracting the values from control wells) in post immunization samples. Following DC vaccination, enhanced responses to ≥ 1 MelAg were detectable in uncultured T cells in 8 of 18 patients (Table II). Five patients showed increased responses to MAGE-3, 4 to MelanA/MART-1, 5 to gp100, and 7 to tyrosinase peptide (Table II and Fig. 4). There was no indication that patients receiving higher DC doses had greater melanoma-specific immunity. Thus, MelAg pulsed CD34-DCs lead to enhancement of MelAg-specific circulating effectors in melanoma patients.

**Table II: Number of peptide-specific IFN-γ ELISPOTS/10⁵ PBMC in pre/post-vaccination samples: circulating effectors (direct ELISPOT, D) and memory T cells (recall ELISPOT, R)**

| ***DC dose*** | ***Pt. I.D.*** | ***Flu*** | ***MAGE*** | ***MART*** | ***Tyr*** | ***Gp100*** | ***# Mel Ags*** |
|---|---|---|---|---|---|---|---|
| 0.1x10⁶/kg | 1 | **D:** 2/19 | 1/8 | 3/3 | 1/6 | 1/8 | *0* |
| | | **R:** 1/276 | 3/40 | 5/59 | 5/45 | 6/40 | *4* |
| | 2 | **D:** 2/16 | 1/4 | 1/0 | 0/2 | 0/8 | *0* |
| | | **R:** 3/12 | 3/2 | 0/14 | 1/9 | 2/14 | *2* |
| | 4 | **D:** 7/39 | 0/36 | 3/25 | 3/27 | 0/33 | *4* |
| | | **R:** ND | ND | ND | ND | ND | |
| | 6 | **D:** 3/77 | 1/2 | 0/1 | 0/2 | 0/2 | *0* |
| | | **R:** ND | 1/3 | ND | 1/7 | 5/13 | *1* |
| | 19 | **D:** 2/10 | 2/6 | 0/1 | 0/4 | 0/1 | *0* |
| | | **R:** 89/164 | 5/20 | 4/14 | 6/8 | 11/14 | *2* |
| 0.25x10⁶/k | 3 | **D:** 5/0 | 0/0 | 0/0 | 0/0 | 0/0 | *0* |
| g | | **R:** 0/0 | 0/0 | 0/0 | 0/0 | 0/0 | *0* |
| | 5 | **D:** 8/31 | 1/23 | 10/13 | 5/24 | 1/25 | *3* |
| | | **R:** 272/1019 | 4/178 | 13/55 | 4/110 | 1/59 | *4* |
| | 9 | **D:** 37/167 | 0/12 | 0/12 | 0/10 | 0/18 | *4* |
| | | **R:** 61/256 | 1/20 | 11/29 | 2/21 | 1/23 | *4* |
| | 16 | **D:** 4/15 | 2/1 | 0/2 | 0/1 | 0/1 | *0* |
| | | 50/225 | 7/38 | 0/47 | 1/45 | 13/48 | *4* |
| 0.5x10⁶/kg | 8 | **D:** 7/19 | 7/20 | 12/39 | 5/15 | 4/5 | *3* |
| | | **R:** ND | ND | ND | ND | ND | |
| | 10 | **D:** 19/89 | 1/4 | 4/0 | 2/0 | 0/0 | *0* |
| | | **R:** 62/730 | 0/0 | 0/15 | 0/54 | 0/35 | *3* |
| | 13 | **D:** 15/13 | 0/0 | 0/0 | 0/0 | 0/0 | *0* |
| | | **R:** ND | ND | ND | ND | ND | *0* |
| | 17 | **D:** 3/18 | 1/9 | 1/12 | 1/10 | 1/7 | *2* |
| | | **R:** 34/441 | 3/37 | 13/64 | 4/51 | 12/44 | *4* |
| 1.0x10⁶/kg | 12 | **D:** 6/21 | 0/3 | 3/8 | 0/13 | 1/4 | *1* |
| | | **R:** 28/100 | 0/0 | 6/20 | 1/36 | 0/36 | *3* |
| | 15 | **D:** 1/3 | 0/0 | 1/0 | 0/1 | 0/10 | *1* |
| | | **R:** 40/155 | 1/4 | 5/6 | 0/6 | 6/70 | *1* |
| | 18 | **D:** 5/13 | 0/2 | 0/1 | 0/1 | 0/1 | *0* |
| | | **R:** 35/43 | 5/18 | 13/9 | 0/2 | 0/16 | *2* |
| | 20 | **D:** 6/25 | 0/0 | 0/0 | 1/0 | 1/2 | *0* |
| | | **R:** 267/326 | 9/5 | 12/10 | 2/14 | 13/22 | *1* |
| | 21 | **D:** 3/17 | 1/15 | 1/9 | 1/10 | 1/10 | *3* |
| | | **R:** 241/473 | 21/37 | 30/44 | 23/20 | 16/36 | *1* |

### Responses in cultured T cells

Next, the capacity of blood CD8 T cells to mount melanoma-specific responses after one-week culture with melanoma peptide-pulsed autologous DCs was determined. Most patients had low levels of MelAg-specific memory cells in pre-immunization samples (except for Patient #21). However, in 14/15 evaluated patients, an increased response to at least one melanoma peptide was found after DC vaccination, (Table II and Fig. 5), and five of these patients responded to all four melanoma peptides (Patients #1, #5, #9, #6, #17).

### Overall response to melanoma antigens

Table III summarizes the DC vaccine, immune responses and clinical outcome (PD=progressive disease; NP=no progression; NE=not evaluable for response). Overall, enhanced immunity to ≥ 1 MelAg by at least one assay was observed in 16 of 18 patients post-DC vaccination (Table III). Of these, enhanced immunity to 2, 3 or all four MelAg was seen by at least one assay in 3, 4, and 6 patients respectively. Thus, vaccination with melanoma-peptide pulsed CD34-DCs leads to enhanced immunity to several MelAg peptides in melanoma patients.

### Clinical outcome

Seven of 17 evaluable patients experienced tumor progression (PD, progression of measurable disease and/or new lesions) (Table I). The remaining ten patients did not progress at this time point (10 weeks from study entry). Among these, three patients (Patient #4, #17 and #21) had neither new lesions nor progression of measurable disease; four patients (Patients #5, #8, #12 and #18) with multiple lesions

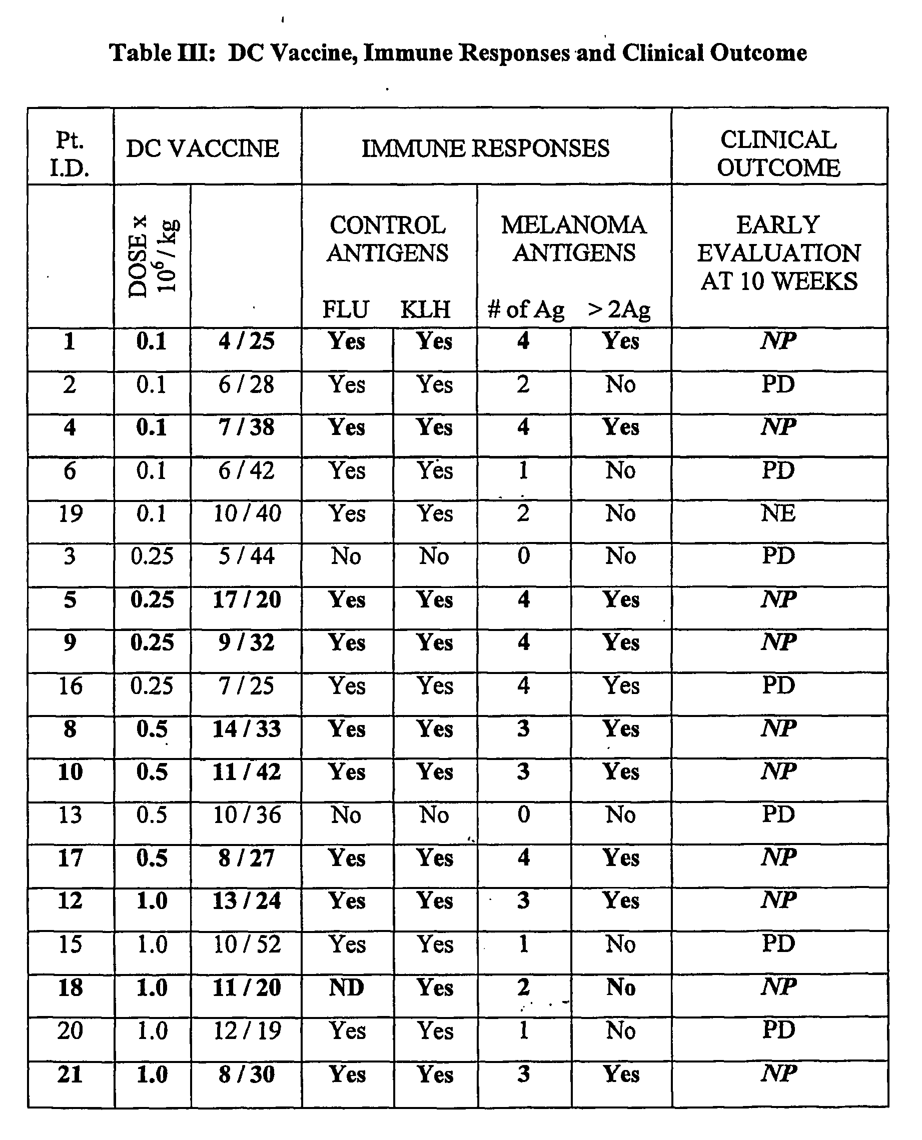

on entry experienced regression at one or more disease sites; and 3 patients (Patients #1, #9, and #10), who had only limited disease on entry, cleared any evidence of disease. Nonprogressing patients received additional DC vaccinations on a subsequent study. Patient #5 received additional immunotherapy at another institution.

### Correlation of Immunologic responses and clinical outcome

Table IV summarizes the correlation between the immune responses in blood and clinical outcome. We first used > 2-fold increase and > 10 antigen specific IFN-γ ELISPOTS in the post-vaccine assays as an indicator of immune response (Dhodapkar, et al. 1999. J Clin Invest 104:173-180; Dhodapkar, et al. 2000. J Clin Invest 105:R9-R14). Two patients (Patient #3 and #13) who failed to respond to either the control or melanoma antigens by any assay, experienced rapid tumor progression and could not complete the planned therapy. Of 17 patients with evaluable disease, 6 of 7 patients who responded to 0, 1 or 2 melanoma antigens had progressive disease upon restaging 10 weeks after study entry (Table III and Table IV). In contrast, tumor progression was seen in only 1 of 10 patients who responded to 3, or all 4 melanoma antigens (p=0.002, Fisher exact test). Regression of > 1 tumor metastases were observed in 7 of these patients.

In order to obtain overall assessment of anti-tumor immunity after the DC vaccine, we integrated data for absolute immune responses to all 4 antigens by both direct and recall assays into a tumor immunity score (from -8.5 to +8.5), as described earlier (Kalbfleisch, J. D. and Prentice, R. L. 1973. Biometrika 60:267-278); and Wittkowski, K. M. 1988. J Am Stat Assoc 83:1163-1170). Among the 17 patients with evaluable disease, tumor immunity was lower among the 7 patients with progressive disease (p=0.043). The tumor immunity score was associated to the clinical outcome. Six of 8 patients with a negative tumor immunity score had progressive disease (Fig. 6). On the other hand, only 1 of the 9 patients with a positive tumor immunity score progressed.

Therefore, both these analyses demonstrated that early clinical outcome after DC vaccination depends upon the elicited immune response.

**Table 4: Summary of Responses**

| RESPONSE TO CONTROL ANTIGENS IN ANY ASSAY | RESPONSE TO > 2 MELANOMA ANTIGENS IN ANY ASSAY | CLINICAL RESPONSE |
|---|---|---|
| No | No | 2 PD |
| Yes | No | 1 SD, 4 PD |
| Yes | Yes | 3 CR, 7 SD, 1 PD |

### EXAMPLE 2: Vaccination with antigen-loaded dendritic cells leads to rapid induction of immune responses in vivo

The analysis of kinetics of KLH-specific immune responses in all samples showed that in a majority of patients, two injections of KLH loaded CD34-DCs were sufficient for induction of significant (p = 0.018 and 0.045 in t-test) proliferative responses at two KLH concentrations (Fig. 7). Table V depicts KLH-specific PBMC proliferation (cpm values) at each time point during the course of vaccination at two KLH concentrations (10 and 1 ng/ml), with the values of spontaneous proliferation subtracted. However, the analysis of each patient revealed in 9/18 patients, KLH-specific proliferation detectable already after the first injection of KLH-pulsed DCs (Table V). Furthermore, in 5 out of these 9 patients, the significant increase (p=0.044 in t-test) in proliferative responses to KLH was seen at the first observation time, i.e., at 5 days after injection (Table V). Among remaining patients responding to KLH, 4 had responses detectable after 2 vaccines, 2 patients after 3 vaccines and 3 patients required 4 injections of KLH-pulsed DCs before significant proliferative responses could be seen.

Seventeen patients received CD34-DCs vaccine where 20% of cells were loaded with Flu-MP peptide. Table VI depicts numbers of Flu-MP peptide-specific IFN-γ ELISPOST/10⁵ PBMC at each time point during the course of vaccination, circulating effectors (i.e. 16 hours assay). Flu specific immunity (defined as at least two-fold increase in Flu-specific IFN-γ ELISPOT at any time point post-immunization) was induced in 15 out of 17 patients and increased with the number of

**Table V: Kinetics of KLH-specific Proliferation**

| Pt # | | d 0 | d 5 | d 14 | d 19 | d28 | d 42 | d 56 | d 70 |
|---|---|---|---|---|---|---|---|---|---|
| KLH ng/ml | | (pre DC) | (DC1) | (DC1) | (DC2) | (DC2) | (DC3) | (DC4) | (DC4) |
| 1: KLH 10 | | 0 | ^{a}2316 | | 1375 | 14054 | 6099 | 14249 | 29711 |
| | KLH 1 | 232 | ^{a}928 | | 0 | 1006 | 2323 | 3884 | 19085 |
| 2: KLH 10 | | 5788 | 3406 | 0 | 0 | ^{b}9067 | 19402 | 56912 | 63937 |
| | KLH 1 | 5 | 0 | 0 | 0 | ^{b}2941 | 6118 | 13492 | 17945 |
| 3: KLH 10 | | 52 | | ^{a}13219 | 0 | 0 | | | |
| | KLH 1 | 0 | | ^{a}6949 | 0 | 0 | | | |
| 4: KLH 10 | | 2949 | | 5569 | | | | | ^{d}82023 |
| | KLH 1 | 254 | | 620 | | | | | ^{d}66616 |
| 5: KLH 10 | | 3878 | | "22298 | 16562 | 28265 | | 75113 | |
| | KLH 1 | 2708 | | ^{a}8227 | 4994 | 20222 | | 58924 | |
| 6: KLH 10 | | 0 | | | | ^{b}43638 | 77341 | 68325 | 16028 |
| | KLH 1 | 0 | | | | ^{b}22746 | 34045 | 60550 | 11880 |
| 8: KLH 10 | | 394 | | ^{a}4179 | 1553 | 15381 | 26805 | 46600 | |
| | KLH 1 | 228 | | ^{a}3538 | 3486 | 3130 | 30223 | 45298 | |
| 9: KLH 10 | | 0 | | 725 | 0 | | ^{d}119226 | 21342 | |
| | KLH 1 | 0 | | 5991 | 0 | | ^{d}72719 | 15509 | |
| 10: KLH 10 | | 671 | ^{a}14945 | 58278 | 155137 | 135487 | 158610 | 151499 | 151468 |
| | KLH 1 | 20 | ^{a}2578 | 25922 | 68001 | 75814 | 90848 | 126231 | 109784 |
| 12: KLH 10 | | 96 | ^{a}7353 | 4600 | | 15673 | 12099 | 43448 | 23673 |
| | KLH 1 | 0 | ^{a}1209 | 891 | | 7885 | 6996 | 34541 | 16593 |
| 13: KLH 10 | | 877 | 1814 | | | 721 | | | ^{d}2088 |
| | KLH 1 | 925 | 343 | | | 469 | | | ^{d}0 |
| 15: KLH 10 | | 89 | ^{a}2637 | | | 13435 | | | 32013 |
| | KLH 1 | 54 | ^{a}867 | | | 3253 | | | 17452 |
| 16: KLH 10 | | 0 | | 382 | 51 | 1764 | ^{d}2554 | 5978 | 603 |
| | KLH 1 | 0 | | 0 | 33 | 0 | ^{d}0 | 1259 | 0 |
| 17: KLH 10 | | 627 | ^{a}5981 | 12418 | 23937 | 81463 | 117865 | 124599 | 154579 |
| | KLH 1 | 0 | ^{a}0 | 2957 | 7437 | 38224 | 67143 | 48599 | 86261 |
| 18: KLH 10 | | 73 | 0 | 157 | 0 | ^{b}5090 | 25998 | 10109 | |
| | KLH 1 | 0 | 0 | 0 | 0 | ^{b}0 | 3267 | 14315 | |
| 19: KLH 10 | | 189 | | 0 | 1259 | | 1816 | ^{d}6636 | 5602 |
| | KLH 1 | 0 | | 0 | 0 | | 1192 | ^{d}1606 | 1578 |
| 20: KLH 10 | | 2039 | | | ^{b}5114 | | | 13851 | |
| | KLH 1 | 507 | | | ^{b}1824 | | | 10639 | |
| 21: KLH 10 | | 3701 | 5572 | ^{a}8179 | 22521 | 32860 | 32054 | 28485 | |
| | KLH 1 | 571 | 1704 | ^{a}3374 | 8071 | 5764 | 10932 | 13134 | |

| | | | | | | | | | |
|---|---|---|---|---|---|---|---|---|---|
| ^{a} indicates responses detectable already after a single DC vaccination. ^{b} indicates responses after 2 vaccines. ^{c} indicates responses after 3 vaccines. ^{d} indicates responses detectable only after 4 vaccines. | | | | | | | | | |

DC injections (Fig. 8). Similarly to KLH, the response to flu-MP peptide was rapidly induced (Fig. 8) and in 8 out of 17 patients was seen already after the first DC injection (Table VI). In 4 out of these 8 patients, considerable increase (p=0.053) in the frequency of Flu-MP specific T cells was observed already after 5 days (Table VI). Among remaining patients responding to Flu-MP, 4 had responses detectable after 2 vaccines, 2 patients after 3 vaccines and only 1 patient required 4 injections of Flu-MP-pulsed DCs before significant responses could be seen.

The analysis of kinetics of immune responses specific to melanoma antigens in all samples showed that for MART-1 as well as for tyrosinase, two injections of peptide-loaded DCs were sufficient to observe significant increase in the frequency of MART-1 responding T cells over the baseline (Fig. 9). In the case of gp100, significant increase could be seen already after a single vaccination while for MAGE-3, 4 injections of peptide-loaded DCs were necessary to observe a significant increase in MAGE-3 specific T cells (Fig. 9). For all peptides, the response increases with repeated vaccinations. Table VII depicts numbers of melanoma peptide-specific IFN-γ ELISPOST/10⁵ PBMC at each time point during the course of vaccination, circulating effectors (i.e., 16 hours assay). As shown in Table VII, seven patients had detectable increase in the frequency of melanoma antigen(s)-specific T cells already after a single DC injection.

Finally, the analysis of kinetics of melanoma-specific responses and comparison between progressive and non-progressive patients revealed a correlation between immune responses and clinical responses, thus corroborating the correlation found using tumor immunity score (Fig. 10).

Thus, vaccination with antigen(s) pulsed CD34-DCs permits rapid induction of immune responses to antigens presented on the vaccine, both control antigens and melanoma antigens. The immune responses correlate with clinical responses and patients who experienced disease progression do not mount melanoma-specific immune responses detectable in the blood.

**Table VI: Kinetics of Flu-MP-specific IFN-γ secreting cells**

| Pt# : ctrl | **d0** | **d5** | **d14** | **d19** | **d28** | **d42** | **d56** | **d70** |
|---|---|---|---|---|---|---|---|---|
| Flu-MP | (pre DC) | (DCI) | (DC1) | (DC2) | (DC2) | (DC3) | (DC4) | (DC4) |
| 1: Ctrl | 3 | 3 | | 3.5 | 2 | **^{d}2.5** | 2 | 3 |
| Flu-MP | 5 | 7.5 | | **4.5** | 6.5 | **^{d}₁₀** | 16 | 22 |
| 2: Ctrl | 2 | 1 | **^{a}1** | 2.5 | 1.5 | 0 | 0 | 1 |
| Flu-MP | **4.5** | 7.5 | **^{a}10** | 9.5 | 23 | 12 | 25 | 17 |
| 3: Ctrl | 0 | 0 | 1 | 0 | 0 | 0 | | |
| Plu-MP | **3.5** | 3 | 0 | 0 | 0 | | | |
| 4: Ctrl | 0.75 | 0 | ^{a}2 | 0 | 0 | 0 | 0.5 | 0 |
| Flu-MP | 8 | 13 | ^{a}18.5 | 7 | 10 | 7 | 17 | 39 |
| 5: Ctrl | 0 | | 0.5 | 0 | 0 | 0.5 | ^{d}0 | |
| Flu-MP | **12** | | **15.5** | 9 | 15.5 | 22.5 | **^{d}31** | |
| 6: Ctrl | 0 | | | | **^{b}0** | 0 | 0 | 0 |
| Flu-MP | **7.5** | | | | **^{b}90** | 94 | 116 | 77.5 |
| 8: Ctrl | 1 | 0 | 7 | 1 | **^{b}0** | 3.5 | 1 | |
| Flu-MP | **11.5** | **2.5** | **12.5** | **7.5** | **^{b}23** | **24** | **20.5** | |
| 9: Ctrl | 3.5 | 3 | **^{a}9.5** | 7 | 5.5 | 0.5 | 6 | |
| Flu-MP | 42 | 41 | **^{a}129** | 165 | 174.5 | 126 | 173 | |
| 10: Ctrl | 0 | 0 | **^{a}0** | 2 | 0 | 0 | 0 | 0 |
| Flu-MP | 20.5 | 30 | **^{a}72** | 90 | 110 | **99.5** | 95 | 90 |
| 12: Ctrl | 3 | **^{a}5.5** | 1 | | 5 | 2 | 8 | 6 |
| Flu-MP | 8 | **^{a}8.5** | 16 | | 21 | 9.5 | 39 | 27 |
| 13: Ctrl | 0 | 0 | 2.5 | | 0 | 0 | 0 | 4 |
| Flu-MP | 16 | **13** | **24** | | **22.5** | **22.5** | **21** | **18** |
| 15: Ctrl | 0 | 1 | 0 | 0 | **^{b}0** | 0 | 0 | |
| Flu-MP | 1 | 1 | 0 | **5** | **^{b}7.5** | 2.5 | 3 | |
| 16: Ctrl | 0 | **^{a}0.5** | 0 | 0 | 0 | 0 | 0 | 0 |
| Flu-MP | 4.5 | **^{a}41.5** | 19 | **19** | **16** | **15** | **15.5** | 15 |
| 17: Ctrl | 0 | 0 | 0 | 0 | **^{b}3** | 4 | 0 | 0 |
| Flu-MP | **4** | **2.5** | **1** | 4.5 | **^{b}15.5** | 24.5 | **17** | 20.5 |
| 19: Ctrl | 2 | 0 | 0 | 0 | 0 | **^{d}0** | 0 | 0 |
| Flu-MP | 7 | 13 | 13 | **10.5** | **9.5** | **^{d}14** | 11.5 | 13 |
| 20: Ctrl | 0 | **^{a}0** | 2 | 1 | 3 | 0 | | |
| Flu-MP | 6 | **^{a}68.5** | 42.5 | **33.5** | 30.5 | 25 | | |
| 21: Ctrl | 0 | **^{a}0** | 1.5 | 1 | 0.5 | 0 | 0 | 0 |
| Flu-MP | 4 | **^{a}18** | 16.5 | 11 | 13 | 9.5 | 14.5 | 17.5 |

| | | | | | | | | |
|---|---|---|---|---|---|---|---|---|
| ^{a} indicates responses detectable already after a single DC vaccination. ^{b} indicates responses after 2 vaccines. ^{c} indicates responses after 3 vaccines. ^{d} indicates responses detectable only after 4 vaccines. | | | | | | | | |

**Table VII: Kinetics of Melanoma-specific IFN-γ Secreting Cells**

| | Pt ID#: | **d 0** | **d5** | **d14** | **d19** | **28** | **d42** | **d56** | **d70** |
|---|---|---|---|---|---|---|---|---|---|
| | Peptide | (pre DC) | (DC1) | (DC 1) | (DC 2) | (DC 2) | (DC 3) | (DC 4) | (DC 4) |
| 1: MART-1 | | 2.5 | 5.5 | | 2.5 | 2 | 2 | 3.5 | **^{a}6** |
| | **Gp 100** | 3.5 | 2 | | 6 | **^{b}8.5** | 4 | 4 | 11 |
| | Tyr | 1.5 | 5 | | 1 | 0 | 3 | 2.5 | **^{d}9** |
| | MAGE-3 | 3 | 2.5 | | 3 | 2 | **^{d}8** | 4.5 | 11 |
| | **Ctrl** | 3 | 3 | | 3.5 | 2 | 2.5 | 2 | 3 |
| *2: MART-1 | | 1.75 | 0 | 4.5 | 0 | 3.5 | 2 | 4 | 1.5 |
| | Gp 100 | 0 | 2 | 2 | 0 | 0 | 1 | **^{d}9.5** | 9 |
| | Tyr | 1 | 0 | 3 | 0 | 1.5 | 0 | **^{d}5.5** | 3.5 |
| | MAGE-3 | 3 | 2 | 0 | 1 | 0 | 0 | 5 | 5 |
| | Ctrl | 2 | 1 | 1 | 2.5 | 1.5 | 0 | 0 | 1 |
| 3: MART-1 | | 0.5 | 0 | 0 | 0 | 0 | 0 | | |
| | Gp 100 | 0 | 0 | 0 | 0 | 0 | 0 | | |
| | Tyr | 0.5 | 0 | 0 | 0 | 0 | 0 | | |
| | MAGE-3 | 0 | 0 | 0 | 0 | 0 | 0 | | |
| | Ctr1 | 0 | 0 | 1 | 0 | 0 | 0 | | |
| *4: MART-1 | | 4 | 6.5 | ^{a}12 | 4 | 0 | 0 | 10 | 25 |
| | Gp 100 | 1 | ^{a}13.5 | 27.5 | 18 | 12 | 6 | 10.5 | 33 |
| | Tyr | 3 | ^{a}7 | 22.5 | 13 | 18 | 5.5 | 8.5 | 27 |
| | MAGE-3 | 0.5 | 3.5 | ^{a}14.5 | 3.5 | 0 | 0 | 16.5 | 36 |
| | Ctrl | 1 | 0 | 2 | 0 | 0 | 0 | 0.5 | 0 |
| *5: MART-1 | | 7 | | 5.5 | 5 | 9.5 | ^{d}14 | 13 | |
| | Gp 100 | 2.5 | | ^{a}8 | 3.5 | 7 | 13.5 | 25 | |
| | Tyr | 5 | | 6 | 4 | 8.5 | 5.5 | ^{d}24 | |
| | MAGE-3 | 1 | | ^{a}5.5 | 3.5 | 6.5 | 4 | 23 | |
| | Ctrl | 0 | | 0.5 | 0 | 0 | 0.5 | 0 | |
| *6: MART-1 | | 0 | | | | ^{b}6.5 | 3 | 7 | 1.5 |
| | Gp 100 | 0 | | | | 3 | ^{d}8.5 | 13.5 | 2 |
| | Tyr | 0 | | | | 1 | 0 | 5.5 | 2.5 |
| | MAGE-3 | 1 | | | | ^{b}5 | 4 | 12.5 | 2.5 |
| | Ctrl | 0 | | | | 0 | 0 | 0 | 0 |
| 8: MART-1 | | 13 | 6 | 17.5 | 12 | 11.5 | ^{d}46 | 40 | |
| | Gp 100 | 7 | 0 | ^{a}14 | 5 | 7.5 | 4.5 | 6.5 | |
| | Tyr | 7 | 2 | ^{a}17 | 7 | 5.5 | 15.5 | 16.5 | |
| | MAGE-3 | 9 | 6 | 6.5 | 3 | 10 | ^{d}22 | 21 | |
| | Ctrl | 1 | 0 | 7 | 1 | 0 | 3.5 | 1 | |
| **9: MART-1 | | 1 | 0.5 | ^{a}17 | 17 | 15 | 12.5 | 18 | |
| | Gp 100 | 1 | 1 | ^{a}17.5 | 19.5 | 12.5 | 6.5 | 24 | |
| | Tyr | 1.25 | 0 | ^{a}16.5 | 21 | 16 | 7.5 | 16.5 | |
| | MAGE-3 | 1.5 | 1 | ^{a}25 | 24.5 | 24 | 16 | 18.5 | |
| | Ctrl | 3.5 | 3 | 9.5 | 7 | 5.5 | 0.5 | 6 | |
| 10: MART-1 | | 4 | 0 | 1 | 0 | 3.5 | 0 | 2 | 1 |
| | Gp 100 | 0.5 | 0 | 0 | 2 | 3.5 | 0 | 0 | 2.5 |
| | Tyr | 2 | 0.5 | 0 | 6.5 | ^{b}7 | 1.5 | 1 | 1 |
| | MAGE-3 | 1.5 | 0 | 0.5 | 0.5 | 1.5 | 1 | 2 | 5 |
| | Ctrl | 0 | 0 | 0 | 2 | 0 | 0 | 0 | 0 |
| 12: MART-1 | | 6 | 4.5 | 4.5 | | 11.5 | 5 | ^{d}23 | 14 |
| | Gp 100 | 1 | ^{a}10 | 7 | | 21 | 8.5 | 10.5 | 10 |
| | Tyr | 4 | 9.5 | 4.5 | | 9 | ^{d}10 | 16.5 | 19 |
| | MAGE-3 | 2.5 | 7.5 | 4 | | 5 | 2.5 | ^{d}3.5 | 9 |
| | Ctrl | 3 | 5.5 | 1 | | 5 | 2 | 8 | 6 |
| 13: MART-1 | | 1.5 | 6.5 | 2 | | 2 | 0 | 0.5 | 4.5 |
| | Gp 100 | 1 | 4.5 | 3 | | 1 | 2 | 0.5 | 5 |
| | Tyr | 2 | 3.5 | 2 | | 1.5 | 3 | 0.5 | 5 |
| | MAGE-3 | 1 | 2.5 | 0 | | 1 | 0 | 1 | 3.5 |
| | Ctrl | 0 | 0 | 2.5 | | 0 | 0 | 0 | 4 |
| * 15: MART-1 | | 1 | 1 | 0 | 2.5 | 3 | 1 | 0 | |
| | Gp 100 | 0.5 | 0 | 0 | 2 | 4.5 | 3.5 | ^{d}10 | |
| | Tyr | 0 | 0 | 0 | 0 | 0.5 | 0 | 1 | |
| | MAGE-3 | 0.5 | 0.5 | 0 | 1.5 | 1 | 0.5 | 0 | |
| | Ctrl | 0 | 1 | 0 | 0 | 0 | 0 | 0 | |
| 16: MART-1 | | 1 | 2.5 | 0 | ^{b}5.5 | 5.5 | 5 | 0 | 2 |
| | Gp 100 | 0 | 1 | 0 | 0 | 3 | 2 | 0 | 1 |
| | Tyr | 0 | 1 | 4 | 4.5 | ^{b}5.5 | 5 | 0 | 3.5 |
| | MAGE-3 | 2 | 4.5 | 3 | 3.5 | ^{b}5.5 | 2.5 | 2 | 1 |
| | Ctrl | 0 | 0.5 | 0 | 0 | 3 | 4 | 0 | 0 |
| 17: MART-1 | | 1 | 0 | 0.5 | 2.5 | 3.5 | ^{d}12.5 | 8.5 | 14 |
| | Gp 100 | 1 | 0 | 0 | 0.5 | 1 | ^{a}12.5 | 3 | 9 |
| | Tyr | 1 | 0 | 1 | 1.5 | 3.5 | ^{d}11 | 3 | 12 |
| | MAGE-3 | 1 | 0 | 0.5 | 2 | 3.5 | 5 | 2 | ^{d}11 |
| | Ctrl | 0 | 0 | 0 | 0 | 3 | 4 | 0 | 0 |
| 18: MART-1 | | 0 | 1.5 | 0 | 0 | 1.5 | 0 | 2 | 1 |
| | Gp 100 | 0 | 0 | 0 | 0 | 0 | 0 | 1.5 | 1 . |
| | Tyr | 0 | 0 | 0 | 0 | 0 | 0 | 1.5 | 4 |
| | MAGE-3 | 2.5 | 2 | 0 | 1.5 | 1.5 | 0 | 2 | ^{d}6.5 |
| | Ctrl | | | | | | | | |
| 19: MART-1 | | 1 | 4.5 | 1.5 | 3.5 | 4.5 | 4 | 1 | 1.5 |
| | Gp 100 | 2.5 | 3 | 1.5 | 0.5 | 0.5 | 0.5 | 1 | 1.5 |
| | Tyr | 2.5 | 3.5 | 1.5 | 0.5 | 0.5 | 1.5 | 1 | 1.5 |
| | MAGE-3 | 2.5 | 0 | 1.5 | 0.5 | 0.5 | 3 | 1 | 2 |
| | Ctrl | 2 | 0 | 0 | 0 | 0 | 0 | 0 | 0 |
| 20: MART-1 | | 1 | ^{a}8.5 | 4 | 6 | 3 | 0 | | |
| | Gp 100 | 1 | 4.5 | 2 | 0 | 1 | 2 | | |
| | Tyr | 1.5 | 5 | 3 | 1 | 0 | 0 | | |
| | MAGE-3 | 0 | 2.5 | 3.5 | 2 | 0 | 0 | | |
| | Ctrl | 0 | 0 | 2 | 1 | 3 | 0 | | |
| 21: MART-1 | | 2 | 0 | ^{a}11.5 | 3.5 | 6 | 10 | 12.5 | 9 |
| | Gp 100 | 2 | 3 | ^{a}7 | 6.5 | 2 | 11.5 | 12 | 10 |
| | Tyr | 2 | 1.5 | 3.5 | 1.5 | 4 | 1.5 | 4.5 | ^{d}10 |
| | MAGE-3 | 3 | 1.5 | 3 | 2.5 | 0.5 | 1.5 | 4.5 | ^{d}10.5 |
| | Ctrl | 0 | 0 | 1.5 | 1 | 0.5 | 0 | 0 | 0 |

| | | | | | | | | | |
|---|---|---|---|---|---|---|---|---|---|
| ^{a} indicates responses detectable already after a single DC vaccination. ^{b} 1indicates responses after 2 vaccines. ^{c} indicates responses after 3 vaccines ^{d} indicates responses detectable only after 4 vaccines. * indicates patients with previous cytokine therapy. ** indicates patient with previous vaccine therapy. | | | | | | | | | |

### EXAMPLE 3: Repeated vaccination with antigen-loaded dendritic cells permits clinical responses.

Twelve (12) patients (5 women and 7 men, median age 57 years (range:43-73 years) with metastatic melanoma (stage IV using the updated American Joint Committee on Cancer Melanoma Staging System) received booster melanoma-antigen loaded CD34-DCs vaccinations after the original four (4) priming DC vaccinations (Patient #1, #4, #8, #9, #10, #12, #13, #16, #17, #18, #19 and #21 in Table I). These DCs were loaded with keyhole limpet hemocyanin (KLH) protein and influenza matrix peptide as well as melanoma antigens. The DC vaccinations were administered subcutaneously at a DC vaccination dose ranging from approximately 0.1 to 1.0 million DC/kilogram/vaccination. Eight (8) patients who experienced stabilization of the disease or a partial response after the first four vaccinations were vaccinated monthly for additional four vaccinations (DCs 5-8) beginning approximately two months after their fourth vaccination and two (2) patients (Patients # 9 and #10) who experienced a complete remission after the first four vaccinations were vaccinated 2 months, 5 months, 9 months, and 15 months after their fourth vaccination. One (1) patient who had experienced a complete remission after the first four vaccinations received a single DC vaccination approximately 8 months after his fourth vaccine (Patient #1). One (1) patient with stable disease after the first four DC vaccinations received two vaccinations at monthly intervals but then had progressive melanoma and was taken off the protocol.

There are four (4) patients alive who remain in complete unmaintained remission. Two (2) additional patients are alive in complete remission following metastatecomy after completing the DC vaccination protocol. One (1) patient who has received additional immunotherapy after completing the protocol remains alive with stable disease 20 months after the last DC vaccination on the protocol. There are five (5) patients treated on the DC protocol who have died of progressive metastatic melanoma. One (1) patient who was not evaluable for response to DC vaccinations relapsed 2 months after completion of the protocol. One (1) patient who experienced a complete remission including complete regression of brain metastatic disease relapsed approximately nine months after her eighth DC vaccination. One (1) patient developed progressive disease after 6 DC vaccinations. Two (2) patients had progressive melanoma 2-4 months after completion of the protocol. There were no significant adverse clinical events associated with DC vaccination. Fig. 11-14 show Kaplan Meier survival curves including disease-free survival from the diagnosis of metastatic melanoma (Fig. 11) and from study entrance (Fig. 12) as well as patient survival from the diagnosis of metastatic melanoma (Fig. 13) and from study entrance (Fig. 14).

Finally, to evaluate the ability of antigen specific T cells to proliferate and differentiate in culture, pre-and post-immunization PBMC were thawed together and co-cultured (2 x 10⁵ cells / well) for 7 days with autologous mature DCs (PBMC:DC ratio 30:1) pulsed with 1 µg/ml peptides. After 7 days, cells were transferred to an ELISPOT plate and cultured overnight with (T: APC ratio 20:1) irradiated (3000 rads) T2 cells with or without specific antigen. Antigen-specific SFCs were calculated after subtracting the background with unpulsed T2 cells. The peptides used in this evaluation included the four peptides that were present on the DC vaccine (vaccine antigens) and two peptides representing tumor antigens (ESO-1 and TERT) that were not presented on the vaccine. As shown in Table VIII, 4 out of 7 melanoma patients who had T cells with specificity for more than two melanoma antigens presented on the vaccine had also detectable T cells specific for other tumor antigens suggesting spreading of the immune response. These four patients are in a complete remission (CR) (Patient #9, #10, #18 and #21). Among the remaining 3 patients, 2 patients experienced initial disease stabilization (SD) followed by progression (PD). One patient with only gp100-specific T cells had a complete disappearance of the melanoma lesions, which was, however, followed by a relapse.

**Table VIII: Summary of Responses after Boosting Immunization**

| RESPONSE TO >2 VACCINE MELANOMA ANTIGENS | RESPONSE TO NON-VACCINE TUMOR ANTIGENS | CLINICAL RESPONSE |
|---|---|---|
| No | No | 1 CR relapse; 1 PD dead |
| No | Yes | 1 SD/PD dead, 1PD dead |
| Yes | No | 1 SD/PD alive; 1SD/PD dead; 1PD |
| Yes | Yes | 4 CR alive |

### EXAMPLE 4: Correlation of spontaneous proliferative PBMC responses and clinical responses.

Analysis of proliferative PBMC responses in samples before onset of treatment and 2-4 weeks after 4^{th} DC vaccination revealed that PBMC from post-DC immunization blood samples, cultured without adding exogenous antigens, proliferate, and in two patients, this proliferation was correlated with the presence of progressive vitiligo (Mel 5 and Mel 8) (Fig. 15). The spontaneous proliferation in samples before start of treatment and at various time points after the 4^{th} vaccine in all patients was determined (Table IX).

As shown in Table IX and in Fig. 16, four out of five patients with index <1 had an early progressive disease, 4 out of 5 patients with index>1 and <10 had clinical response at 10 weeks after study entry (no progression), and all patients with index>10 had clinical response. This was confirmed by analyzing spontaneous proliferation in blood samples at two different time points post-immunization (Fig. 17) and the data obtained with same samples at two different labs (Fig. 17). Furthermore, as shown in Fig. 18, the index of spontaneous proliferation, i.e. without exogenous antigens, was significantly correlated with the index of antigen-specific proliferation, i.e., KLH indicating that the level of spontaneous proliferation may be predictive of the antigen-specific proliferative responses (p=0.003 in simple regression). Finally, the proliferating cells are CD4+ T cells (Fig. 19). Furthermore, the activation of the immune system induced by DC vaccination could be best illustrated by the increase in proliferative responses to Tetanus Toxoid as shown in Table X for pre and post-vaccination samples.

DC vaccine was not loaded with TT, yet in 10 out of 14 evaluated, patients' TT responses were higher in the post-immunization samples (range of fold-increase: 1.6 to 82, median 1.7 fold.

Thus, DC vaccination leads to a very strong immunization against both identified (i.e. delivered on vaccine) as well as unknown antigens. The level of blood immune responses correlates with and is predictive of clinical responses.

**Table IX: Spontaneous^{a} proliferation before and after DC vaccination in patients with metastatic melanoma**

| | Pre-DCs^{b} | Post-DCs^{c} | Index | Clinical response^{d} |
|---|---|---|---|---|
| MEL 01 | 390 | 256 | 0.65 | NP |
| MEL 02 | 160 | 41 | 0.26 | PD |
| MEL 04 | 239 | 2276 | 9.5 | NP |
| MEL 05 | 3448 | 26780 | 7.8 | NP |
| MEL 06 | 264 | 219 | 0.83 | PD |
| MEL 08 | 94 | 4139 | 43.8 | NP |
| MEL 09 | 7236 | 10303 | 1.42 | NP |
| MEL 10 | 1914 | 70890 | 37 | NP |
| MEL 12 | 1182 | 12332 | 10.43 | NP |
| MEL 13 | 550 | 188 | 0.34 | PD |
| MEL 15 | 105 | 644 | 6.1 | PD |
| MEL 16 | 107 | 120 | 1.1 | PD |
| MEL 17 | 512 | 27526 | 53.8 | NP |
| MEL 18 | 269 | 1196 | 4.4 | NP |
| MEL 19 | 107 | 316 | 2.96 | NE |
| MEL 21 | 148 | 2442 | 16.5 | NP |

| | | | | |
|---|---|---|---|---|
| ^{a} Spontaneous=in the absence of exogenous antigen. ^{b} PBMC are cultured for 5 days, and proliferation is measured by thymidine incorporation (cpm). ^{c} At various time points after the 4^{th} DC vaccine ^{d} At 10 weeks after study entry: NP, no progression; PD, progressive melanoma; NE, non-evaluable for clinical response | | | | |

**Table X: Proliferation to Tetanus Toxoid before and after DC vaccination in patients with metastatic melanoma^{a}**

| | Pre-DCs^{b} | Post-DCs^{e} | Fold increase | Clinical response^{d} |
|---|---|---|---|---|
| MEL 01 | 1145 | 272 | 0.24 | NP |
| MEL 04 | 7374 | 80909 | 11 | NP |
| MEL 05 | 20630 | 58232 | 2.8 | NP |
| MEL 06 | 36543 | 3618 | 0.09 | PD |
| MEL 08 | 19997 | 34160 | 1.7 | NP |
| MEL 09 | 9250 | 12934 | 1.4 | NP |
| MEL 10 | 55104 | 206755 | 3.7 | NP |
| MEL 12 | 18209 | 31482 | 1.7 | NP |
| MEL 13 | 2143 | 694 | 0.32 | PD |
| MEL 16 | 1074 | 796 | 0.74 | PD |
| MEL 17 | 27166 | 50547 | 1.9 | NP |
| MEL 18 | 174 | 14307 | 82 | NP |
| MEL 19 | 10750 | 17036 | 1.6 | NE |
| MEL 21 | 3707 | 13893 | 3.7 | NP |

| | | | | |
|---|---|---|---|---|
| ^{a} DCs are not loaded with TT for vaccination. ^{b} PBMC are cultured for 5 days and proliferation is measured by thymidine incorporation (cpm). ^{c} At various time points after the 4^{th} DC vaccine. ^{d} At 10 weeks after study entry: NP, no progression; PD, progressive melanoma; NE, non-evaluable. | | | | |

To summarize, the invention pertains to the following embodiments:
1. In one embodiment, the invention is a method for treating malignancy in a mammal comprising administering to said mammal in need of such treatment an effective amount of a vector for vaccination comprising antigen-presenting cells loaded with at least two or more agents selected from the group consisting of tumor antigens and tumor antigen derived peptides.
2. In another embodiment, the invention is a method for treating malignancy in a mammal comprising administering to said mammal in need of such treatment an effective amount of a vector for vaccination comprising at least two or more tumor antigens or tumor antigen derived peptides, wherein said vaccine targets antigen-presenting cells in said mammal.
3. In another embodiment, the invention is a method for treating malignancy in a mammal comprising administering to said mammal in need of such treatment an effective amount of two or more vectors for vaccination wherein each vector comprises antigen-presenting cells loaded with one unique agent selected from the group consisting of tumor antigens and tumor antigen derived peptides.
4. In another embodiment, the invention is a method for treating malignancy in a mammal comprising administering to said mammal in need of such treatment an effective amount of two or more vectors for vaccination wherein each vector comprises one unique agent selected from the group consisting of tumor antigens and tumor antigen derived peptides, and wherein said vector targets antigen-presenting cells in said mammal.
5. In another embodiment, the invention is a method for preventing malignancy in a mammal comprising administering to said mammal an effective amount of a vector for vaccination comprising antigen-presenting cells loaded with at least two tumor antigens or tumor antigen derived peptides.
6. In another embodiment, the invention is a method for preventing malignancy in a mammal comprising administering to said mammal an effective amount of a vector for vaccination comprising at least two or more tumor antigens or tumor antigen derived peptides, wherein said vector targets antigen-presenting cells in said mammal.
7. In another embodiment, the invention is a method for preventing malignancy in a mammal comprising administering to said mammal an effective amount of two or more vectors for vaccination wherein each vector comprises antigen-presenting cells loaded with one unique agent selected from the group consisting of tumor antigens and tumor antigen derived peptides.
8. In another embodiment, the invention is a method for preventing malignancy in a mammal comprising administering to said mammal an effective amount of two or more vectors for vaccination wherein each vector comprises one unique agent selected from the group consisting of tumor antigens and tumor antigen derived peptides, wherein said vector targets antigen-presenting cells in said mammal.
9. In another embodiment, in the method of embodiments 1, 3, 5 or 7, said antigen-presenting cells are dendritic cells.
10. In another embodiment, in the method of embodiment 9, said antigen-presenting cells are dendritic cells derived from CD34+ hematopoietic progenitors.
11. In another embodiment, in the method of embodiment 10, said dendritic cells are generated by culturing CD34 hematopoietic progenitors in a tissue culture in the absence of any foreign serum component.
12. In another embodiment, in the method of embodiment 10, said dendritic cells are generated by culturing CD34 hematopoietic progenitors in a tissue culture in the presence of autologous serum, said autologous serum not being heat inactivated before addition to said tissue culture.
13. In another embodiment, the method of embodiments 1, 2, 3, 4, 5, 6, 7 or 8 further comprises antigen-presenting cells loaded with control antigens.
14. In another embodiment, the method of embodiment 9 further comprises antigen-presenting cells loaded with control antigens.
15. In another embodiment, the method of embodiments 10, 11 or 12 further comprises antigen-presenting cells loaded with control antigens.
16. In another embodiment, the invention is a method for predicting the *in vivo* anti-tumor efficacy of a vaccine, said vaccine comprising antigen-presenting cells loaded with at least two or more tumor antigens, tumor antigen derived peptides or control antigens, comprising
   isolating at a first time point a first sample of peripheral blood mononuclear cells from a patient before administering said vaccine to form pre-immunization cells;
   storing said pre-immunization cells under conditions that preserve the proliferative integrity of said pre-immunization cells;
   immunizing said patient with said vaccine;
   isolating at at least one subsequent time point a post-immunization sample of peripheral blood mononuclear cells from said patient to form post-immunization cells;
   separately and simultaneously culturing said pre-immunization and post-immunization cells in the absence of exogenous antigens;
   measuring the amount of proliferation of said pre-immunization cells and said post-immunization cells;
   comparing the amount of proliferation of said post-immunization cells to the amount of proliferation of said pre-immunization cells;
   wherein a ratio of the amount of proliferation of post-immunization cells to the amount of proliferation of pre-immunization cells being greater than or equal to one is positively predictive of effective anti-tumor activity of said vaccine in said patient.
17. In another embodiment, the invention is a method for predicting the *in vivo* anti-tumor efficacy of a vaccine, said vaccine comprising two or more vectors wherein each vector comprises antigen-presenting cells loaded with one unique agent selected from the group consisting of tumor antigens, tumor antigen derived peptides and control antigens, comprising
   isolating at a first time point a first sample of peripheral blood mononuclear cells from a patient before administering said vaccine to form pre-immunization cells;
   storing said pre-immunization cells under conditions that preserve the proliferative integrity of said pre-immunization cells;
   immunizing said patient with said vaccine;
   isolating at at least one subsequent time point a post-immunization sample of peripheral blood mononuclear cells from said patient to form post-immunization cells;
   separately and simultaneously culturing said pre-immunization and post-immunization cells in the absence of exogenous antigens;
   measuring the amount of proliferation of said pre-immunization cells and said post-immunization cells;
   comparing the amount of proliferation of said post-immunization cells to the amount of proliferation of said pre-immunization cells;
   wherein a ratio of the amount of proliferation of post-immunization cells to the amount of proliferation of pre-immunization cells being greater than or equal to one is positively predictive of effective anti-tumor activity of said vaccine in said patient.
18. In another embodiment, the invention is a method for predicting the *in vivo* anti-tumor efficacy of a vaccine, said vaccine comprising at least two or more vectors of vaccination wherein each vector comprises one unique agent selected from the group consisting of tumor antigens, tumor antigen derived peptides and control antigens, wherein said vectors target antigen-presenting cells in said mammal, comprising
   isolating at a first time point a first sample of peripheral blood mononuclear cells from a patient before administering said vaccine to form pre-immunization cells;
   storing said pre-immunization cells under conditions that preserve the proliferative integrity of said pre-immunization cells;
   immunizing said patient with said vaccine;
   isolating at at least one subsequent time point a post-immunization sample of peripheral blood mononuclear cells from said patient to form post-immunization cells;
   separately and simultaneously culturing said pre-immunization and post-immunization cells in the absence of exogenous antigens;
   measuring the amount of proliferation of said pre-immunization cells and said post-immunization cells;
   comparing the amount of proliferation of said post-immunization cells to the amount of proliferation of said pre-immunization cells;
   wherein a ratio of the amount of proliferation of post-immunization cells to the amount of proliferation of pre-immunization cells being greater than or equal to one is positively predictive of effective anti-tumor activity of said vaccine in said patient.
19. In another embodiment, the invention is a method for predicting the *in vivo* anti-tumor efficacy of a vaccine, said vaccine comprising a vector for vaccination comprising at least two or more tumor antigens, tumor antigen derived peptides or control antigens, wherein said vector targets antigen-presenting cells in said mammal, comprising
   isolating at a first time point a first sample of peripheral blood mononuclear cells from a patient before administering said vaccine to form pre-immunization cells;
   storing said pre-immunization cells under conditions that preserve the proliferative integrity of said pre-immunization cells;
   immunizing said patient with said vaccine;
   isolating at at least one subsequent time point a post-immunization sample of peripheral blood mononuclear cells from said patient to form post-immunization cells;
   separately and simultaneously culturing said pre-immunization and post-immunization cells in the absence of exogenous antigens;
   measuring the amount of proliferation of said pre-immunization cells and said post-immunization cells;
   comparing the amount of proliferation of said post-immunization cells to the amount of proliferation of said pre-immunization cells;
   wherein a ratio of the amount of proliferation of post-immunization cells to the amount of proliferation of pre-immunization cells being greater than or equal to one is positively predictive of effective anti-tumor activity of said vaccine in said patient.
20. In another embodiment, the invention is a method for predicting the *in vivo* anti-tumor efficacy of a vaccine, said vaccine comprising antigen-presenting cells loaded with at least two or more agents selected from the group consisting of tumor antigens, tumor antigen derived peptides and control antigens, comprising
   immunizing at a first time point a patient with said vaccine;
   measuring at at least one post-immunization time point said patient's immunologic response to said agents;
   wherein a positive immunologic response to at least two agents is positively predictive of effective anti-tumor activity of said vaccine in said patient.
21. In another embodiment, the invention is a method for predicting the *in vivo* anti-tumor efficacy of a vaccine, said vaccine comprising two or more vectors wherein each vector comprises antigen-presenting cells loaded with one unique agent selected from the group consisting of tumor antigens, tumor antigen derived peptides and control antigens, comprising
   immunizing at a first time point a patient with said vaccine;
   measuring at at least one post-immunization time point said patient's immunologic response to said agents;
   wherein a positive immunologic response to at least two agents is positively predictive of effective anti-tumor activity of said vaccine in said patient.
22. In another embodiment, the invention is a method for predicting *the in vivo* anti-tumor efficacy of a vaccine, said vaccine comprising a vector for vaccination comprising at least two or more agents selected from the group consisting of tumor antigens, tumor antigen derived peptides and control antigens, comprising
   immunizing at a first time point a patient with said vaccine;
   measuring at at least one post-immunization time point said patient's immunologic response to said agents;
   wherein a positive immunologic response to at least two agents is positively predictive of effective anti-tumor activity of said vaccine in said patient.
23. In another embodiment, the invention is a method for predicting the *in vivo* anti-tumor efficacy of a vaccine, said vaccine comprising two or more vectors wherein each vector one unique agent selected from the group consisting of tumor antigens, tumor antigen derived peptides and control antigens, comprising
   immunizing at a first the point a patient with said vaccine;
   measuring at at least one post-immunization time point said patient's immunologic response to said agents;
   wherein a positive immunologic response to at least two agents is positively predictive of effective anti-tumor activity of said vaccine in said patient.
24. In another embodiment, in the method of embodiments 16, 17, 20 or 21, said antigen-presenting cells are dendritic cells.
25. In another embodiment, in the method of embodiment 24, said antigen-presenting cells are dendritic cells derived from CD34+ hematopoietic progenitors.
26. In another embodiment, in the method of embodiment 25, said dendritic cells are generated by culturing CD34 hematopoietic progenitors in a tissue culture in the absence of any foreign serum component.
27. In another embodiment, in the method of embodiment 25, said dendritic cells are generated by culturing CD34 hematopoietic progenitors in a tissue culture in the presence of autologous serum, said autologous serum not being heat inactivated before addition to said tissue culture.
28. In another embodiment, the invention is a method for predicting a subject's clinical outcome from two or more immune responses, comprising the steps of:
   (a) identifying the subject as a separate group to be compared with previously treated subjects grouped by their clinical response;
   (b) partially ordering all profiles by determining for all pairs of profiles the order of a first profile compared to a second profile as (a) superior, (b) inferior, (c) equal, or (d) undecided, wherein a partial ordering comprises the first profile superior if for each variable the first profile is superior or equal, and for at least one variable, the first profile is superior;
   (c) computing all rankings compatible with all pairwise partial orderings, wherein among two ordered subjects the subject being superior is assigned the higher rank;
   (d) generating a score for each profile by averaging across the rankings; and
   (e) predicting the clinical outcome by selecting the group whose average score most closely resembles the score of the subject's profile.
29. In another embodiment, the invention is a method for generating CD34 dendritic cells, comprising culturing CD34 hematopoietic progenitors in a tissue culture in the absence of any foreign serum component.
30. In another embodiment, the invention is a method for generating CD34 dendritic cells, comprising culturing CD34 hematopoietic progenitors in a tissue culture in the presence of autologous serum, said autologous serum not being heat inactivated before addition to said tissue culture.
31. In another embodiment, the invention is a vaccine comprising antigen-presenting cells loaded with at least two or more agents selected from the group consisting of tumor antigens, tumor antigen derived peptides and control antigens, wherein said antigen-presenting cells are CD34 dendritic cells isolated from a tissue culture of CD34 hematopoietic progenitors wherein said a tissue culture lacks any foreign serum component.
32. In another embodiment, the invention is a vaccine comprising antigen-presenting cells loaded with at least two or more agents selected from the group consisting of tumor antigens, tumor antigen derived peptides and control antigens, wherein said antigen-presenting cells are CD34 dendritic cells isolated from a tissue culture of CD34 hematopoietic progenitors wherein said a tissue culture comprises autologous serum, said autologous serum not being heat inactivated before addition to said tissue culture.
33. In another embodiment, the invention is a vaccine comprising two or more vectors for vaccination wherein each vector comprises antigen-presenting cells loaded with one unique agent selected from the group consisting of tumor antigens, tumor antigen derived peptides and control antigens, wherein said antigen-presenting cells are CD34 dendritic cells isolated from a tissue culture of CD34 hematopoietic progenitors wherein said a tissue culture lacks any foreign serum component.
34. In another embodiment, the invention is a vaccine comprising two or more vectors for vaccination wherein each vector comprises antigen-presenting cells loaded with one unique agent selected from the group consisting of tumor antigens, tumor antigen derived peptides and control antigens, wherein said antigen-presenting cells are CD34 dendritic cells isolated from a tissue culture of CD34 hematopoietic progenitors wherein said a tissue culture comprises autologous serum, said autologous serum not being heat inactivated before addition to said tissue culture.
35. In another embodiment, the invention is a vaccine comprising at least two or more vectors selected from the group consisting of tumor antigens, tumor antigen derived peptides and control antigens targeting antigen-presenting cells in a mammal.

## Claims

1. A vaccine comprising antigen-presenting cells loaded with at least two or more agents selected from the group consisting of tumor antigens and tumor antigen derived peptides and with control antigens.

2. The vaccine of claim 1, wherein said antigen-presenting cells are dendritic cells.

3. The vaccine of claim 2, wherein said dendritic cells are derived from CD34 hematopoietic progenitors.

4. The vaccine of claim 3, wherein said dendritic cells are generated by culturing CD34 hematopoietic progenitors in a tissue culture in the absence of any foreign serum component.

5. The vaccine of claim 3, wherein said dendritic cells are generated by culturing CD34 hematopoietic progenitors in a tissue culture in the presence of autologous serum, said autologous serum not being heat inactivated before addition to said tissue culture.

6. The vaccine of claims 1 to 5, wherein said control antigens are selected from the group consisting of keyhole limpet hemocyanin, viral antigens, flu matrix peptide and protein antigens.

7. The vaccine of claims 1 to 6, wherein the tumor antigens are melanoma antigens selected from the group consisting of melanaA/MART-1, tyrosinase, MAGE-3 and gp100.

8. A vaccine of claims 1 to 7 for use in the treatment of malignancy in a mammal.

9. The vaccine for use of claim 8, wherein said malignancy is metastatic melanoma.

10. The vaccine for use of claim 8 or 9, wherein the treatment is by repeated administration of the vaccine.

11. The vaccine for use of claim 8 or 9, wherein the treatment involves an induction phase and a consolidation phase.

12. The vaccine for use of claim 11, wherein the induction phase comprises administering the vaccine every other week.

13. The vaccine for use of claim 11, wherein the consolidation phase comprises administering the vaccine on a monthly basis.

14. A vaccine of claims 1 to 7 for use in the prevention of malignancy in a mammal.

15. The vaccine for use of claim 14, wherein said malignancy is metastatic melanoma.
